# EUROPEAN PATENT SPECIFICATION

(11) **EP 1 137 644 B1**
(45) Date of publication and mention of the grant of the patent: **10.09.2003**
(21) Application number: 99957263.9
(22) Date of filing: 03.12.1999
(51) Int. Cl.: C07D 307/87, A61K 31/343

(54) **BENZOFURAN DERIVATIVES, THEIR PREPARATION AND USE**
BENZOFURANDERIVATE, IHRE HERSTELLUNG UND VERWENDUNG
DERIVES DE BENZOFURANE, LEUR PREPARATION ET LEUR APPLICATION

(30) Priority: 08.12.1998 US 111360 P; 09.12.1998 DK 163198
(43) Date of publication of application: 04.10.2001
(73) Proprietor: H. Lundbeck A/S, 2500 Valby-Copenhagen (DK)
(72) Inventor: ANDERSEN, Kim, DK-2830 Virum (DK); ROTTLÄNDER, Mario, DK-2500 Valby (DK); BÖGESÖ, Klaus, Peter, DK-2970 Hörsholm (DK); PEDERSEN, Henrik, DK-2700 Brönshöj (DK); RUHLAND, Thomas, DK-2500 Valby (DK); DANCER, Robert, DK-1961 Frederiksberg C (DK)
(74) Representative: Meidahl Petersen, John
(86) International application number: DK9900676
(87) International publication number: WO00034263

(56) References cited:
- WO-A1-95/18118
- DE-A1- 2 657 013
- GB-A- 1 173 312

## Description

The present invention relates to novel benzofuran derivatives potently binding to the 5-HT_{1A} receptor, pharmaceutical compositions containing these compounds and the use thereof for the treatment of certain psychiatric and neurological disorders. Many of the compounds of the invention are also potent serotonin reuptake inhibitors and are considered to be particularly useful for the treatment of depression.

Clinical studies of known 5-HT_{1A} partial agonists such as e.g. buspirone, ipsapirone and gepirone have shown that 5-HT_{1A} partial agonists are useful in the treatment of anxiety disorders such as generalised anxiety disorder, panic disorder, and obsessive compulsive disorder (Glitz, D. A., Pohl, R., *Drugs* 1991, *41*, 11). Preclinical studies indicate that full agonists also are useful in the treatment of the above mentioned anxiety related disorders (Schipper, *Human Psychopharm.,* 1991, 6, S53).

There is also evidence, both clinical and preclinical, in support of a beneficial effect of 5-HT_{1A} partial agonists in the treatment of depression as well as impulse control disorders and alcohol abuse (van Hest, *Psychopharm.,* 1992, *107*, 474; Schipper et al, *Human Psychopharm.,* 1991, 6, S53; Cervo et al, *Eur. J. Pharm*., 1988, *158*, 53; Glitz, D. A., Pohl, *R., Drugs* 1991, 41, 11; Grof et al., *Int. Clin. Psychopharmacol*. 1993, *8,* 167-172; Ansseau et al., *Human Psychopharmacol*. 1993, 8, 279-283).

5-HT_{1A} agonists and partial agonists inhibit isolation-induced aggression in male mice indicating that these compounds are useful in the treatment of aggression (Sanchéz et al, *Psychopharmacology*, 1993, *110*, 53-59).

Furthermore, 5-HT_{1A} agonists have been reported to show activity in animal models predictive for antipsychotic effects (Wadenberg and Ahlenius, *J. Neural. Transm*., 1991, *83*, 43; Ahlenius, *Pharmacol*. *& Toxicol*., 1989, *64*, 3; Lowe et al., *J. Med. Chem*., 1991, *34*, 1860; New et al., *J. Med. Chem*., 1989, 32, 1147; and Martin et al., *J. Med. Chem*., 1989, 32, 1052) and may therefore be useful in the treatment of psychotic disorders such as schizophrenia. Recent studies also indicate that 5-HT_{1A} receptors are important in the serotonergic modulation of haloperidol-induced catalepsy (Hicks, *Life Science* 1990, *47*, 1609) suggesting that 5-HT_{1A} agonists are useful in the treatment ofthe side effects induced by conventional antipsychotic agents such as e.g. haloperidol.

5-HT_{1A} agonists have shown neuroprotective properties in rodent models of focal and global cerebral ischaemia and may, therefore, be useful in the treatment of ischaemic disease states (Prehn , *Eur. J. Pharm*. 1991, *203*,213).

Pharmacological studies have been presented which indicate that 5-HT_{1A} antagonists are useful in the treatment of senile dementia (Bowen et al., Trends Neur. Sci. 1992, *15*, 84).

An overview of 5-HT_{1A} antagonists and proposed potential therapeutic targets for these antagonists based upon preclinical and clinical data are presented by Schechter et al., *Serotonin*, 1997, Vol.2, Issue 7. It is stated that 5-HT_{1A} antagonists may be useful in the treatment of schizophrenia, dementia associated with Alzheimer's disease, and in combination with SSRI antidepressants also to be useful in the treatment of depression.

Both in animal models and in clinical trials it has been shown that 5-HT_{1A} agonists exert antihypertensive effects *via* a central mechanism (Saxena and Villalón, *Trends Pharm. Sci.* 1990, *11*, 95; Gillis *et al, J. Pharm. Exp. Ther.* 1989, *248,* 851). 5-HT_{1A} ligands may, therefore, be beneficial in the treatment of cardiovascular disorders.

5-HT reuptake inhibitors are well known antidepressant drugs and useful for the treatment of panic disorders and social phobia.

The effect of combined administration of a compound that inhibits serotonin reuptake and a 5-HT_{1A} receptor antagonist has been evaluated in several studies (Innis, R.B. et al., *Eur. J. Pharmacol*., **1987,** *143, p 195-204* and Gartside, S.E., *Br. J. Pharmacol*. **1995,** *115, p 1064-1070*, Blier, P. *et al, Trends Pharmacol: Sci*. **1994,** *15*, 220). In these studies it was found that 5-HT_{1A} receptor antagonists would abolish the initial brake on 5-HT neurotransmission induced by the serotonin reuptake inhibitors and thus produce an immediate boost of 5-HT transmission and a more rapid onset of therapeutic action.

Several patent applications have been filed which cover the use of a combination of a 5-HT_{1A} antagonist and a serotonin reuptake inhibitor for the treatment of depression (see EP-A2-687 472 and EP-A2-714 663).

Accordingly, agents acting on the 5-HT_{1A} receptor, both agonists and antagonists, are believed to be of potential use in the therapy of psychiatric and neurological disorders and thus being highly desired. Furthermore, antagonists at the same time having potent serotonin reuptake inhibition activity may be useful for the treatment of depression.

Compounds different from the invention are disclosed in WO 95/18118. The compounds are described as useful in the treatment of various diseases in the CNS as well as in cardiovascular disorders.

DE 2657013 describes phthalane derivatives different from the compounds of the invention. These compounds are described as anti-depressants.

### Summary of the Invention

It has now been found that compounds of a certain class of benzofuran derivatives bind to the 5-HT_{1A} receptor with high affinities. Furthermore, it has been found that many of these compounds have potent serotonin reuptake inhibition activity.

Accordingly, the present invention relates to novel compounds of the general Formula I: 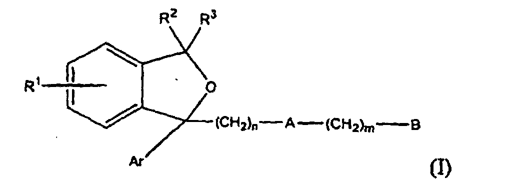 wherein
R¹ is hydrogen, halogen, trifluoromethyl, trifluoromethylsulfonyloxy, C₁₋₆ alkyl, C₂₋₆ alkenyl, C₂₋₆ alkynyl, C₃₋₈ cycloalkyl, C₁₋₆ alkoxy, hydroxy, formyl, acyl, amino, C₁₋₆ alkylamino, C₂₋₁₂ dialkylamino, acylamino, C₁₋₆ alkoxycarbonylamino, aminocarbonylamino, C₁₋₆ alkylaminocarbonylamino, C₂₋₁₂ dialkylaminocarbonylamino, nitro, cyano, COOH, or COO-C₁₋₆ alkyl;
R² and R³ are each independently selected from hydrogen, trifluoromethyl, C₁₋₆ alkyl, C₂₋₆ alkenyl, C₂₋₆ alkynyl, C₃₋₈ cycloalkyl and C₁₋₆ alkoxy;
n is 1, 2, 3, 4 or 5;
m is 0 or 1;

A is selected from the following groups: 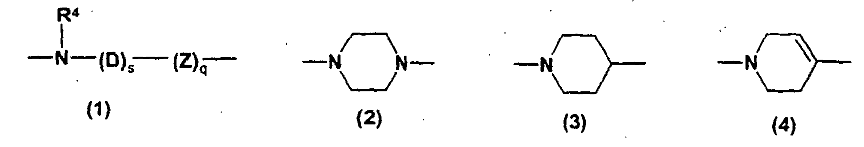 wherein
Z is O or S;
s is 0 or 1;
q is 0 or 1;

R⁴ is hydrogen, C₁₋₆-alkyl, C₂₋₆-alkenyl, C₂₋₆-alkynyl, C₁₋₆-alkyl-Aryl, or C₁₋₆-alkyl-O-Aryl,
D is a spacer group selected from branched or straight chain C₁₋₆-alkylene, C₂₋₆-alkenylene and C₂₋₆-alkynylene;
B is a group selected from a group of formula (II), (III), and (IV) 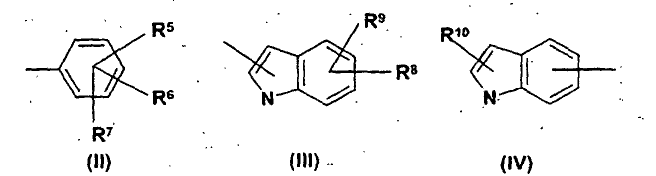 wherein R⁵, R⁶, R⁷, R⁸, R⁹ and R¹⁰ are each independently selected among the R¹ substituents;
or R⁸ and R⁹ together form a fused 5- or 6-membered ring optionally containing further heteroatoms;
or two of the groups of R⁵, R⁶ and R⁷ are linked together thereby forming a ―O―(CH₂)ₚ―O― -bridge wherein p is 1 or 2;
Ar and Aryl are independently selected from the group consisting of phenyl, 2-thienyl, 3-thienyl, 2-furanyl, 3-furanyl, 2-pyrimidyl, 1-indolyl, 2-indolyl, 3-indolyl, indol-2-on-1-yl, indol-2-on-3-yl, 2- or 3-benzofuranyl, 2- or 3-benzothiophenyl, 1-naphthyl or 2-naphthyl, each optionally substituted with halogen, C₁₋₆ alkyl, C₁₋₆ alkoxy, C₁₋₆ alkylthio, hydroxy, C₁₋₆ alkylsulfonyl, cyano, trifluoromethyl, trifluoromethylsulfonyloxy, C₃₋₈ cycloalkyl, C₃₋₈ cycloalkyl-C₁₋₆ alkyl, nitro, amino, C₁₋₆ alkylamino, C₂₋₁₂ dialkylamino, acylamino or alkylenedioxy;
its enantiomers, and pharmaceutically acceptable acid addition salt thereof.

In one embodiment of the invention A is a group of formula (1) and the other substituents are as defined above.

In another embodiment of the invention A is a group of formula (2) and the other substituents are as defined above.

In a third embodiment of the invention A is a group of formula (3) and the other substituents are as defined above.

In a fourth embodiment of the invention A is a group of formula (4) and the other substituents are as defined above.

Thus in a preferred embodiment of the invention A is a group of formula (1) and R⁴ is methyl, ethyl, propyl, prop-2-en-1-yl, 2-furylmethyl, or 2-phenoxyethyl; q = 0; or A is a group of formula (1) and Z is O and the other substituents are as defined above.

In a further embodiment of the invention, B is a group of formula (II), preferably a alkoxysubstituted phenyl, a benzodioxan group or a 1,2-methylenedioxybenzene group and the other substituents are as defined above.
In a further embodiment of the invention, B is a group of formula (III), preferably a 3-indolyl group and the other substituents are as defined above.

In a further embodiment of the invention, B is a group of formula (III), preferably a 3-indolyl group and the substituents R⁸ and R⁹ are preferably selected from hydrogen, methyl, fluoro, chloro, bromo, iodo, *t*-butyl or *i*-propyl in the 5-position; or fluoro, chloro or carboxy in the 7-position; or by 5,7-difluoro, 4-fluoro-7-methyl or 4-chloro-7-methyl; or the two substituents together form a pyridyl ring fused to the 3-indolyl.

In a further embodiment of the invention, B is a group of formula (IV) and the other substituents are as defined above.

Ar is preferably phenyl or phenyl substituted with halogen or CF₃, most preferably substituted with F or Cl in the 4-position or Cl or CF₃ in the 3-position.

R¹ is preferably H, CN or F in the 5-position of the isobenzofuran group.

R² and R³ are preferably selected from hydrogen or methyl.

n is preferably 2, 3 or 4.

m is preferably 0.

In a preferred embodiment of the invention n = 2, 3 or 4; R² and R³ are both hydrogen; R¹ is H, CN or F in the 5-position of the isobenzofuran group; and Ar is phenyl which may be substituted with F or Cl in the 4-position or with Cl or CF₃ in the 3-position and the other substituents are as defined above.

In another preferred embodiment of the invention, A is a group of formula (1); q = 0; R⁴ is methyl; D is propylene; m = 0; and B is a 1,4-benzodioxan group of Formula (II) attached in the 5-position and the other substituents are as defined above.

In another preferred embodiment of the invention, A is a group of formula (1); R⁴ is CH₃ or prop-2-en-1-yl; n = 3; D is ethylene or propylene; and B is a phenyl group wherein at least one substituent is OMe and the other substituents are as defined above.

In a further embodiment of the invention, A is a group of formula (1); q is 0; R⁴ is methyl, ethyl, propyl, 2-propen-1-yl, 2-furylmethyl or 2-phenoxyethyl; D is ethylene, propylene or butylene; m = 0; and B is a 3-indolyl group of Formula (III) and the other substituents are as defined above.

In another preferred embodiment of the invention, A is a group of formula (2) or (3); n = 3; m = 0; and B is an 4- or 5-indolyl-group of Formula (IV) wherein R¹⁰ is hydrogen; R¹ is CN in the 5-position of the isobenzofuran and Ar is 4-Fluorophenyl and the other substituents are as defined above.

The invention also relates to a pharmaceutical composition comprising a compound of formula (I) or a pharmaceutically acceptable salt thereof and at least one pharmaceutically acceptable carrier or diluent.

In a further embodiment, the invention relates to the use of a compound of formula (I) or a pharmaceutically acceptable acid addition salt thereof for the preparation of a medicament for the treatment of a disorder or disease responsive to the effect of 5-HT_{1A} receptors.

In particular, the invention relates to the use of a compound according to the invention or a pharmaceutically acceptable acid addition salt thereof for the preparation of a medicament for the treatment of depression, psychosis, anxiety disorders, panic disorder, obsessive compulsive disorder, impulse control disorder, alcohol abuse, aggression, ischaemia, senile dementia, cardiovascular disorders or social phobia.

The compounds of the invention have high affinity for the 5-HT_{1A} receptor. Accordingly, the compounds of the invention are considered useful for the treatment of depression, psychosis, anxiety disorders, such as generalised anxiety disorder, panic disorder, and obsessive compulsive disorder, impulse control disorder, alcohol abuse, aggression, ischaemia, senile dementia, cardiovascular disorders and social phobia.

Due to their combined antagonism of 5-HT_{1A} receptors and serotonin reuptake inhibiting effect, many of the compounds of the invention are considered particularly useful as fast onset of action medicaments for the treatment of depression. The compounds may also be useful for the treatment of depression in patients who are resistant to treatment with currently available antidepressants.

### Detailed Description of the Invention

Some of the compounds of general Formula I may exist as optical isomers thereof and such optical isomers are also embraced by the invention.
The term C₁₋₆ alkyl refers to a branched or unbranched alkyl group having from one to six carbon atoms inclusive, such as methyl, ethyl, 1-propyl, 2-propyl, 1-butyl, 2-butyl, 2-methyl-2-propyl and 2-methyl-1-propyl.

Similarly, C₂₋₆ alkenyl and C₂₋₆ alkynyl, respectively, designate such groups having from two to six carbon atoms, inclusive.

Halogen means fluoro, chloro, bromo, or iodo.

The term C₃₋₈ cycloalkyl designates a monocyclic or bicyclic carbocycle having three to eight C-atoms, such as cyclopropyl, cyclopentyl, cyclohexyl, cycloheptyl, and cyclooctyl.

The terms C₁₋₆ alkoxy, C₁₋₆ alkylthio, C₁₋₆ alkylsulfonyl, designate such groups in which the alkyl group is C₁₋₆ alkyl as defined above.

Acyl means -CO-alkyl wherein the alkyl group is C₁₋₆ alkyl as defined above.

C₁₋₆ alkylamino means -NH-alkyl, and C₂₋₁₂ dialkylamino means -N-(alkyl)₂ where the alkyl group is C₁₋₆ alkyl as defined above.

Acylamino means -NH-acyl wherein acyl is as defined above.

C₁₋₆ alkoxycarbonylamino means alkyl-O-CO-NH- wherein the alkyl group is C₁₋₆ alkyl as defined above.

C₁₋₆ alkylaminocarbonylamino means alkyl-NH-CO-NH- wherein the alkyl group is C₁₋₆ alkyl as defined above.

C₂₋₁₂ dialkylaminocarbonylamino means (alkyl)₂-N-CO-NH- wherein the alkyl group is C₁₋₆ alkyl as defined above.

Exemplary of organic acid addition salts according to the invention are those with maleic, fumaric, benzoic, ascorbic, succinic, oxalic, bis-methylenesalicylic, methanesulfonic, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzenesulfonic, and theophylline acetic acids, as well as the 8-halotheophyllines, for example 8-bromotheophylline. Exemplary of inorganic acid addition salts according to the invention are those with hydrochloric, hydrobromic, sulfuric, sulfamic, phosphoric, and nitric acids. The acid addition salts of the invention are preferably pharmaceutically acceptable salts formed with non-toxic acids.

Furthermore, the compounds of this invention may exist in unsolvated as well as in solvated forms with pharmaceutically acceptable solvents such as water, ethanol and the like. In general, the solvated forms are considered equivalent to the unsolvated forms for the purposes of this invention.

Some of the compounds of the present invention contain chiral centres and such compounds exist in the form of isomers (e.g. enantiomers). The invention includes all such isomers and any mixtures thereof including racemic mixtures.

Racemic forms can be resolved into the optical antipodes by known methods, for example, by separation of diastereomeric salts thereof with an optically active acid, and liberating the optically active amine compound by treatment with a base. Another method for resolving racemates into the optical antipodes is based upon chromatography on an optically active matrix. Racemic compounds of the present invention can thus be resolved into their optical antipodes, e.g., by fractional crystallisation of d- or 1-(tartrates, mandelates, or camphorsulphonate) salts for example. The compounds of the present invention may also be resolved by the formation of diastereomeric derivatives.

Additional methods for the resolution of optical isomers, known to those skilled in the art, may be used. Such methods include those discussed by J. Jaques, A. Collet, and S. Wilen in "Enantiomers, Racemates, and Resolutions", John Wiley and Sons, New York (1981).

Optically active compounds can also be prepared from optically active starting materials.

The compounds of the invention can be prepared by one of the following methods comprising:
a) alkylating an amine of formula 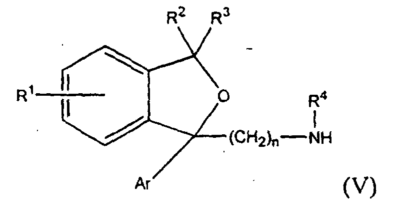 wherein R¹, R², R³, R⁴, n and Ar are as defined above with an alkylating agent of formula G-(D)ₛ-(Z)_{q}-(CH₂)ₘ-B wherein D, Z, m, s, q and B are as defined above and G is a suitable leaving group such as halogen, mesylate, or tosylate;
b) alkylating an amine of formula H-A-(CH₂)ₘ-B wherein A, m and B are as defined above with an alkylating agent of formula 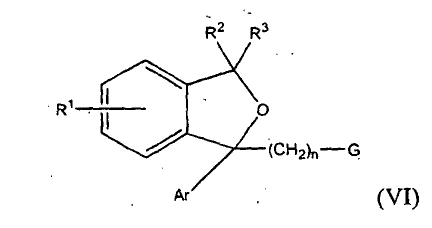 wherein R¹, R², R³, n and Ar are as defined above and G is a suitable leaving group such as halogen, mesylate, or tosylate;
c) reductive alkylation of an amine of formula 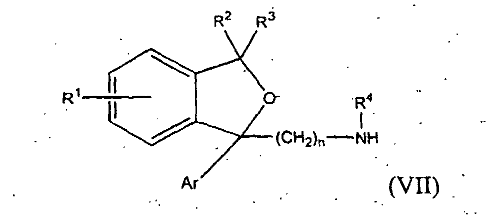 wherein R¹, R², R³, R⁴, n and Ar are as defined above with an aldehyde of formula 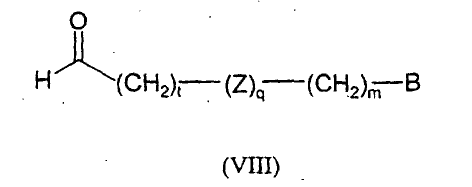 wherein Z, m, q and B are as defined above and t is 1-5;
d) reducing an amide of formula 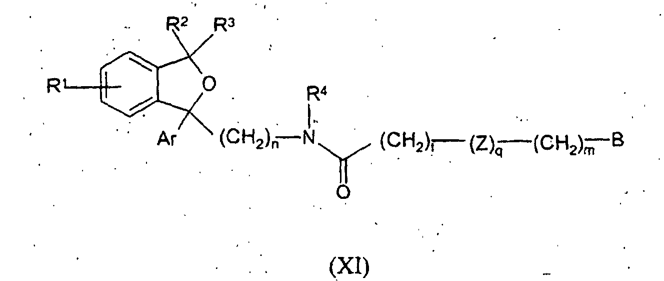 wherein R¹, R², R³, R⁴, n, q, Ar, Z, m and B are as defined above and t is 1-5;
e) releasing final product by the means of Hofmann elimination from a resin of formula 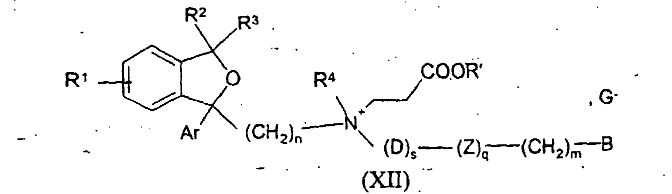 wherein R¹, R², R³, R⁴, n, s, q, Ar, D, Z, m and B are as defined above, G is as defined above; and HOR' is a hydroxy substituted resin such as cross linked hydroxymethylpolystyrene or Wang resin.
f) reacting a compound of the formula 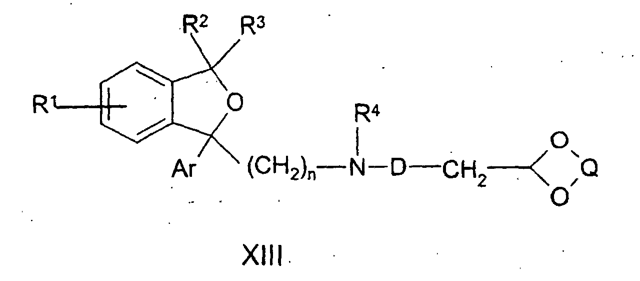
wherein R¹, R², R³, R⁴, Ar, D and N are as defined above; (OH)₂Q is a diol such as substituted ethylene glycol or propylene glycol, or a polymer bound diol, with a hydrazine of formula 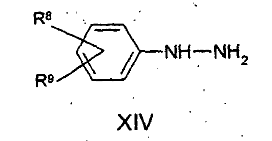 wherein R⁸ and R⁹ is as defined above, using Lewis acids as catalyst.

The alkylations according to Methods a and b are generally performed by boiling the reactants under reflux or by heating them at a fixed temperature in a suitable solvent such as acetone, methyl isobutyl ketone, tetrahydrofuran, dioxane, ethanol, 2-propanol, ethyl acetate, N,N-dimethylformamide, dimethyl sulfoxide or 1-methyl-2-pyrrolidinone in the presence of a base such as triethylamine or potassium carbonate. Amines of formula V are prepared by means of demethylation according to the method described by Bigler et al, Eur. J: Med. Chem. Chim. Ther, 1977, 12, 289-295, or by the methods outlined in examples 14 and 15. The starting materials used in example 14 were prepared as described in example 9 or from readily available compounds by standard methods. The enantiomers of 1-[3-(dimethylamino)propyl]-1-(4-fluorophenyl)-1,3-dihydroisobenzofuran-5-carbonitrile used as starting material for the demethylation are prepared as described in EP patent No. 347066. The alkylating agents of formula G-(D)ₛ-(Z)_{q}-(CH₂)ₘ-B are commercially available, prepared by methods obvious to the chemist skilled in the art or prepared as exemplified in Examples 5-8. Ethyl 1,4-benzodioxan-5-carboxylate used as starting material in Example 5 is prepared by methods obvious to the chemist skilled in the art from the corresponding carboxylic acid prepared according to literature (Fuson et al., J. Org. Chem., 1948, 13, 489). Alkylating agents of formula VI are prepared from the corresponding dimethylamine (Formula VI: G = N(Me)₂) as exemplified in example 9. The secondary amines of formula H-A-(CH₂)ₘ-B are commercially available, prepared by methods obvious to the chemist skilled in the art or prepared according to literature procedures. 1-(2-methoxyphenyl)piperazine is prepared according to Pollard et al., J. Org. Chem., 1958, 23, 1333. [2-(2-Methoxyphenoxy)ethyl]methylamine and [2-(3-methoxyphenoxy)ethyl]-methylamine are prepared as exemplified in Examples 7 and 10 using commercially available 2-methoxyphenoxyacetic acid and 3-methoxyphenoxyacetic acid, respectively, as starting materials.

The reductive alkylations according to method c and d are performed according to standard literature methods using NaCNBH₃, NaBH₄ or NaBH(OAc)₃ as reducing agent in a suitable solvent.

The reductions according to Methods e and f are generally performed by use of LiAlH₄, AlH₃ or diborane in an inert solvent such as tetrahydrofuran, dioxane, or diethyl ether at room temperature or at a slightly elevated temperature.

The release of final products by means of Hofmann elimination in Method g is generally performed by the use of an organic base such as triethylamine or diisopropylethylamine in an aprotic organic solvent such as dichloromethane, toluene or N,N-dimethylformamide. The polymer of formula XII is prepared in a synthesis sequence as exemplified in Example 4 and described in the following. The starting acryl ester resin (CH₂CHC(O)OR') is prepared according to literature procedures (Brown et al., J. Am. Chem. Soc., 1997, 119, 3288-95) by acylation of commercially available hydroxy substituted resins such as cross linked hydroxymethylpolystyrene or Wang resin with acryloyl chloride. Secondary amines of formula H₂N-D-Z-(CH₂)ₘ-B are introduced by Michael addition in an organic solvent such as N,N-dimethylformamide at ambient temperature. The secondary amines used are either commercially available, prepared by methods obvious to the chemist skilled in the art or prepared according to literature procedures. 3-(2-Methoxyphenyl)propylamine is prepared according to Leeson et al., J. Med. Chem. 1988, 31, 37-54, 3-(3-methoxyphenyl)propylamine according to Meise et al. Liebigs Ann. Chem., 1987, 639-42, 3-(2-methoxyphenoxy)propylamine according to Augsein et al., J. Med. Chem., 1965, 8, 356-67, 3-(3-methoxyphenoxy)propylamine according to Bremner et al., Aust. J. Chem. 1984, 37, 129-41, 2-benzyloxyethylamine according to Harder et al. Chem. Ber. 1964, 97, 510-19, 2-(1*H*-indolyl-3-yl)ethylamine according to Nenitzescu et al., Chem. Ber., 1958, 91, 1141-45 and 3-(1*H*-indolyl-3-yl)propylamine according to Jackson et al., J. Am. Chem. Soc., 1930, 52, 5029. The second diversifying group is introduced by means of alkylation with an agent of formula VI by boiling the reactants under reflux or by heating them at a fixed temperature in a suitable solvent such as tetrahydrofuran, dioxane, ethanol, 2-propanol, ethyl acetate, N,N-dimethylformamide, dimethyl sulfoxide or 1-methyl-2-pyrrolidinone in the presence of a soluble base such as diisopropylethylamine or triethylamine, or by means of reductive alkylation with an aldehyde of formula IX using standard solid phase synthesis literature methods using NaCNBH₃, NaBH₄ or NaBH(OAc)₃ as reducing agent in a suitable solvent. The third diversifying group was introduced by means of quartemisation using an alkylating agent of formula R⁴-G in an organic solvent such as tetrahydrofuran, dioxane, ethanol, 2-propanol, ethyl acetate, N,N-dimethylformamide, dimethyl sulfoxide or 1-methyl-2-pyrrolidinone at ambient temperature giving resins of formula XII.

The indole formation according to method h is performed by the reaction of acetals of formula XIII with aryl hydrazines of formula XIV resulting in the corresponding hydrazones, which subsequently are converted into indoles by means of the Fischer indole synthesis. The synthesis sequence is preferably performed as a one-pot procedure using a Lewis acid catalysts, preferably zinc chloride or boron fluoride, or protic acids, preferably sulfuric acid or phosphoric acid, in a suitable solvent such as acetic acid or ethanol at an elevated temperature. Acetals of formula XIII are prepared by alkylation of secondary amines of formula V with acetals of formula XV 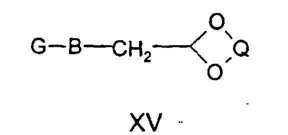 using the conditions described above for methods a and b. Alternatively, the acetals of formula XIII are prepared by alkylation of acetals of formula XVI 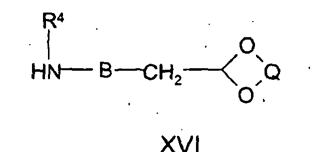 with an alkylating agent of formula VI using the conditions described above for methods a and b. The acetals of formula XVI are prepared by reaction of acetals of formula XV with primary amines of formula NH₂R⁴ using standard conditions.
Polymer bound acetals of formula XV is prepared by reaction of aldehydes of formula G-B-CH₂CHO with commercially available 2,2-dimethyl-1,3-dioxolan-4-yl-methoxymethyl polystyrene in a suitable solvent such as toluene, using p-toluenesulfonic acid as catalyst at elevated temperature. 4-Chlorobutanal, 5-chloropentanal, and 6-chlorohexanal were prepared in analogy to the method described by Normant et al., Tetrahedron 1994, 50 (40), 11665.

### Examples

Melting points were determined on a Büchl SMP-20 apparatus and are unconnected. Mass spectra were obtained on a Quattro MS-MS system from VG Biotech, Fisons Instruments. The MS-MS system was connected to an HP 1050 modular HPLC system. A volume of 20-50 µl of the sample (10 µg/ml) dissolved in a mixture of 1% acetic acid in acetonitril/water 1:1 was introduced via the autosampler at a flow of 30 µl/min into the Electrospray Source. Spectra were obtained at two standard sets of operating conditions. Analytical LC-MS data were obtained on a PE Sciex API 150EX instrument equipped with IonSpray source and Shimadzu LC-8A/SLC-10A LC system. The LC conditions (50 X 4.6 mm YMC ODS-A with 5 µm particle size) were linear gradient elution with water/acetonitrile/trifluoroacetic acid (90:10:0.05) to water/acetonitrile/trifluoroacetic acid (10:90:0.03) in 7 min at 2 mL/min. Purity was determined by integration of the UV trace (254 nm). The retention times Rₜ are expressed in minutes.
One set to obtain molecular weight information (MH+) (21 eV) and the other set to induce fragmentation patterns (70 eV). The background was subtracted. The relative intensities of the ions are obtained from the fragmentation pattern. When no intensity is indicated for the Molecular Ion (MH+), this ion was only present under the first set of operating conditions. Preparative LC-MS-separation was performed on the same instrument. The LC conditions (50 X 20 mm YMC ODS-A with 5 µm particle size) were linear gradient elution with water/acetonitrile/trifluoroacetic acid (80:20:0.05) to water/acetonitrile/trifluoroacetic acid (10:90:0.03) in 7 min at 22.7 mL/min. Fraction collection was performed by split-flow MS detection.
'H NMR spectra were recorded at 500.13 MHz on a Bruker Avance DRX500 instrument or at 250.13 MHz on a Bruker AC 250 instrument. Deuterated chloroform (99.8%D) or dimethyl sulfoxide (99.9%D) were used as solvents. TMS was used as internal reference standard. Chemical shift values are expressed in ppm-values. The following abbreviations are used for multiplicity of NMR signals: s=singlet, d=doublet, t=triplet, q=quartet, qui=quintet, h=heptet, dd=double doublet, dt=double triplet, dq=double quartet, tt=triplet of triplets, m=multiplet. NMR signals corresponding to acidic protons are generally omitted. Content of water in crystalline compounds was determined by Karl Fischer titration. Standard workup procedures refer to extraction with the indicated organic solvent from proper aqueous solutions, drying of combined organic extracts (anhydrous MgSO₄ or Na₂SO₄), filtering and evaporation of the solvent *in vacuo*. For column chromatography silica gel of type Kieselgel 60, 230-400 mesh ASTM was used.

### Example 1

(+)-1-[3-[[4-(1,4-Benzodioxan-5-yl)butyl]methylamino]propyl]-1-(4-fluorophenyl)-1,3-dihydroisobenzofuran-5-carbonitrile (**1a**). A mixture of 5-(4-bromobutyl)-1,4-benzodioxane (1.5 g, 5.5 mmol), (+)-1-[3-(methylamino)propyl]-1-(4-fluorophenyl)-1,3-dihydroisobenzofuran-5-carbonitrile (2.2. g, 5.5 mmol), potassium carbonate (3.0 g, 22 mmol), and methyl isobutyl ketone (150 mL) was boiled under reflux for 16 h. After cooling to room temperature the organic phase was washed with water (150 mL), the solvents evaporated *in vacuo* and the remaining oil purified by column chromatography (ethyl acetate/heptane/triethylamine 75:20:5) affording 2.0 g (73%) of the title compound as an oil: [α]²²_{D} + 8.93° (c 0.5; CH₃OH). ¹H NMR (CDCl₃) δ 1.25-1.35 (m, 1H), 1.40-1.60 (m, 5H), 2.05-2.30 (m, 9H), 2.55 (t, 2H), 4.20-4.30 (m, 4H), 5.10-5.20 (m, 2H), 6.65-6.75 (m, 3H), 7.00 (t, 2H), 7.35 (d,1H), 7.40 (dd, 2H), 7.50 (s, 1H), 7.60 (d, 1H); MS m/z 501 (MH⁺, 100), 262 (27), 149 (77), 109 (52).

The following compounds were prepared analogously:
(+)-1-[3-[[3-(1,4-Benzodioxan-5-yl)propyl]methylamino]propyl]-1-(4-fluorophenyl)-1,3-dihydroisobenzofuran-5-carbonitrile oxalate (**1b**): mp 114-16°C (ethyl acetate);[α]²²_{D} + 8.96° (c 1.0; CH₃OH); ¹H NMR (DMSO-*d*_{*6*}) δ 1.35-1.45 (m, 1H), 1.45-1.55 (m, 1H), 1.80 (m, 2H), 2.20-2.30 (m, 2H), 2.45-2.55 (m, 2H), 2.60 (s, 3H), 2.90 (m, 2H), 2.95 (m, 2H), 4.20-4.30 (m, 4H), 5.20 (m, 2H), 6.65-6.75 (m, 3H), 7.10-7.20 (m, 2H), 7.55-7.60 (m, 2H), 7.70-7.80 (m, 1H), 7.80-7.95 (m, 2H); MS m/z 488 (MH+, 100), 262 (33), 149 (52), 109 (55).

1-[3-[[2-(1,4-Benzodioxan-5-yl)ethyl]methylamino]propyl]-1-(4-fluorophenyl)-1,3-dihydroisobenzofuran-5-carbonitrile oxalate (**1c**): mp 118-20°C (ethyl acetate); ¹H NMR (DMSO-*d*_{*6*}) δ 1.40-1.70 (m, 2H), 2.25 (t, 2H), 2.70 (s, 3H), 2.75-2.90 (m, 2H), 2.90-3.15 (m; 4H), 4.15-4.30 (m, 4H), 5.20 (m, 2H), 6.65-6.80 (m, 3H), 7.20 (t, 2H), 7.60 (dd, 2H), 7.70-7.85 (m, 3H); MS m/z 473 (MH+, 64), 323 (13), 262 (24), 163 (100), 109 (25).

1-[3-[[1,4-Benzodioxan-5-ylmethyl]methylamino]propyl]-1-(4-fluorophenyl)-1,3-dihydroisobenzofuran-5-carbonitrile oxalate (**1d**): mp 160-62 °C (acetone/methanol); ¹H NMR (DMSO-*d*_{*6*}) δ 1.40-1.70 (m, 2H), 2.25 (t, 2H), 2.60 (s, 3H), 2.90 (t, 2H), 4.00 (s, 2H), 4.20-4.30 (m, 4H), 5.20 (m, 2H), 6.80-7.00 ( m, 3H), 7.15 (t, 2H), 7.50 - 7.65 (dd, 2H), 7.70-7.85 (m, 3H); MS m/z 459 (MH+, 7), 109 (100).

### Example 2

1-(4-Fluorophenyl)-1-[3-[4-(2-methoxyphenyl)piperazinyl]propyl]-1,3-dihydroisobenzofuran-5-carbonitrile (**2a**). A mixture of 1-(3-chloropropyl)-1-(4-fluorophenyl)-1,3-dihydroisobenzofuran-5-carbonitrile (2.5 g, 7.9 mmol), 1-(2-methoxyphenyl)piperazine (2.0 g, 10.4 mmol), potassium carbonate (3 g, 22 mmol) and methyl isobutyl ketone (200 mL) was boiled under reflux for 16 h. After cooling to room temperature the organic phase was washed with water (200 mL), the solvents were evaporated *in vacuo* and the remaining oil purified by column chromatography (ethyl acetate/heptane/triethylamine 75:20:5). The title compound crystallised from diethyl ether 1.5 g (40 %): mp 147-49 °C; ¹H NMR (DMSO-*d*_{*6*}) δ 1.30-1.65 (m, 2H), 2.10-2.30 (m, 2H), 2.40 (t, 2H), 2.50-2.70 (m, 4H), 2.90-3.20 (m, 4H), 3.85 (s, 3H), 5.20 (m, 2H), 6.70-7.10 (m, 6H), 7.30-7.55 (m, 4H), 7.60 (d, 1H); MS m/z, 472 (MH+, 100), 262 (14), 109 (19).

The following compounds were prepared analogously:
1-(4-Fluorophenyl)-1-[3-[[2-(2-methoxyphenoxy)ethyl]methylamino]propyl]-1,3-dihydroisobenzofuran-5-carbonitrile (**2b**): (oil) ¹H NMR (CDCl₃) δ 1.30-1.40 (m, 1H), 1.40-1.55 (m, 1H), 2.10-2.20 (m, 2H), 2.25 (s, 3H), 2.40-2.45 (t, 2H), 2.70-2.80 (m, 2H), 3.70 (s, 3H), 4.05 (t, 2H), 5.15 (m, 2H), 6.85-7.00 (m, 6H), 7.30-7.45 (m, 3H), 7.50 (s, 1H), 7.55 (d, 1H).

1-(4-Fluorophenyl)-1-[3-[[2-(3-methoxyphenoxy)ethyl]methylamino]propyl]-1,3-dihydroisobenzofuran-5-carbonitrile (**2c**): (oil) ¹H NMR (CDCl₃) δ 1.30-1.40 (m, 1H), 1.40-1.55 (m, 1H), 2.10-2.20 (m, 2H), 2.25 (s, 3H), 2.40 (t, 2H), 2.70-2.75 (m, 2H), 3.70 (s, 3H), 4.00 (t, 2H), 5.15 (m, 2H), 6.40-6.55 (m, 3H), 7.00 (t, 2H), 7.20 (t, 1H), 7.35 (d, 1H), 7.40 (dd, 2H), 7.50 (s, 1H), 7.55 (d, 1H).

(*S*)-1-[3-[[4-(1*H*-Indol-3-yl)butyl]methylamino]propyl]-1-(4-fluorophenyl)-1,3-dihydroisobenzofuran-5-carbonitrile (**2d**): LC/MS (m/z) 482 (MH⁺), Rt = 4.24, purity: 84 %.

1-[3-[[4-(1*H*-Indol-3-yl)butyl]methylamino]propyl]-1-phenyl-1,3-dihydroisobenzofuran (**2e**): LC/MS (m/z) 439 (MH⁺), Rt = 4.33, purity: 77 %.

(*S*)-1-[3-[[3-(1*H*-Indol-3-yl)propyl]methylamino]propyl]-1-(4-fluorophenyl)-1,3-dihydroisobenzofuran-5-carbonitrile (**2f**): LC/MS (m/z) 468 (MH⁺), Rt = 4.11, purity: >99 %.

1-[3-[[3-(1*H*-Indol-3-yl)propyl]methylamino]propyl]-1-phenyl-1,3-dihydroisobenzofuran (**2g**): LC/MS (m/z) 425 (MH⁺), Rt = 4.15, purity: >99 %.
5-[3-[[3-(1-Phenyl-1,3-dihydroisobenzofuran-1-yl)propyl]methylamino]propyl]-1,4-benzodioxane (**2h**): LC/MS (m/z) 444 (MH⁺), Rt = 4.12, purity: 97 %. 5-[3-[[3-[1-(3-Chlorophenyl)-1,3-dihydroisobenzofuran-1-yl]propyl]methylamino]propyl]-1,4-benzodioxane (**2i**): LC/MS (m/z) 478 (MH⁺), Rt = 4.45, purity: 93 %.
5-[3-[[3-[1-(4-Fluorophenyl)-1,3-dihydroisobenzofuran-1-yl]propyl]methylamino]propyl]-1,4-benzodioxane (**2j**): LC/MS (m/z) 462 (MH⁺), Rt = 4.21, purity: 93 %.
5-[3-[[3-[1-(3-Trifluoromethylphenyl)-1,3-dihydroisobenzofuran-1-yl]propyl]methylamino]propyl]-1,4-benzodioxane (**2k**): LC/MS (m/z) 512 (MH⁺), Rt = 4.59, purity: 90 %.
1-[3-[[3-(1,4-Benzodioxan-5-yl)propyl]methylamino]propyl]-1-(4-chlorophenyl)-1,3-dihydroisobenzofuran-5-carbonitrile (**2l**): LC/MS (m/z) 503 (MH⁺), Rt = 4.59, purity: >99 %.
1-[3-[4-(1*H*-Indol-4-yl)piperazinyl]propyl]-1-(4-fluorophenyl)-1,3-dihydroisobenzofuran-5-carbonitrile (**2m**): LC/MS (m/z) 481 (MH⁺), Rt = 5.61, purity: 97 %.
1-[3-[4-(1*H*-Indol-5-yl)piperazinyl]propyl]-1-(4-fluorophenyl)-1,3-dihydroisobenzofuran-5-carbonitrile (**2n**): LC/MS (m/z) 481 (MH⁺), Rt = 5.69, purity: 94 %.
1-[3-[4-(6-chloro-1*H*-Indol-3-yl)piperidinyl]propyl]-1-(4-fluorophenyl)-1,3-dihydroisobenzofuran-5-carbonitrile (**2o**): LC/MS (m/z) 514 (MH⁺), Rt = 6.38, purity: 96 %.

### Example 3

5-[3-[[3-[-5-Fluoro-1-(4-fluorophenyl)-1,3-dihydroisobenzofuran-1-yl]propyl]methylamino]propyl]-1,4-benzodioxane oxalate (**3**). A solution of 3-(1,4-benzodioxan-5-yl)propionic acid (0.8 g, 3.8 mmol), thionyl chloride (1 mL, 13.7 mmol) and one droplet of N,N-dimethylformamide in dichloromethane (30 mL) was boiled under reflux for 2 h. The volatile solvents were evaporated *in vacuo* and the remaining oil was dissolved in dichloromethane (30 mL). The resulting solution was added to a solution [3-[-5-fluoro-1-(4-fluorophenyl)-1,3-dihydroisobenzofuran-1-yl]propyl]methylamine (3.0 g, 10 mmol) and triethylamine (10 mL) in dichloromethane (100 mL). After stirring for 16 h the volatile solvents were evaporated *in vacuo* and the remaining oil was purified by column chromatography (ethyl acetate/heptane 75:25) affording 1.4 g of crude amide which was used without further purification.
To a solution of the amide (1.4 g, 2.8 mmol) in tetrahydrofuran (200 mL) was added lithium aluminum hydride (1.0 g, 2.6 mmol). After boiling of the resulting mixture under reflux for 3 h, the reaction mixture was cooled to 0 °C and carefully treated with water (1 mL) and 4 N aqueous sodium hydroxide (1 mL). The resulting mixture was filtered and dried (Na₂SO₄). Evaporation of the volatile solvents afforded the title compound as an oil which was precipitated as its oxalate in acetone 0.9 g (19%): mp 131-33 °C; ¹H NMR (DMSO-*d*_{*6*}) δ 1.35-1.45 (m, 1H), 1.45-1.55 (m, 1H), 1.75-1.80 (m, 2H), 2.10-2.25 (m, 2H), 2.50-2.55 (m, 2H), 2.60 (s, 3H), 2.90 (t, 2H), 2.95 (t, 2H), 4.20-4.25 (m, 4H), 5.10 (m, 2H), 6.65-6.75 (m, 3H), 7.10-7.15 (m, 4H), 7.45-7.60 (m, 3H); MS m/z, 480 (MH+, 100), 225 (34), 109 (51).

### Example 4

1-[3-[[2-(1*H*-Indol-3-yl)ethyl]methylamino]propyl]-1-(4-fluorophenyl)-1,3-dihydroisobenzofuran-5-carbonitrile (**4a**). To a suspension of acryl ester Wang resin (CH₂CHC(O)OR', HOR' = Wang resin) (loading 1.0 mmol/g) (300 mg, 0.30 mmol) (prepared from Wang resin (Loading 1.09 mmol/g, 200-400 36-75 µm (mesh), 1% divinylbenzene) in analogy with the procedure described for the preparation of acryl ester hydroxymethyl polystyrene by Brown et al., J. Am. Chem. Soc., 1997, 119, 3288-95) in N,N-dimethylformamide (1.5 mL) was added a solution of 2-(1*H*-indolyl-3-yl)ethylamine (96 mg, 0.60 mmol) in N,N-dimethylformamide (1.5 mL). After stirring of the resulting suspension at room temperature for 16 h, the resin was filtered off and subsequently washed with 0.3 M diisopropylethylamine in N,N-dimethylformamide (3 X 2.5 mL), methanol (2 X 2.5 mL) and dichloromethane (2 X 2.5 mL).
To a suspension of the resulting resin in acetonitrile (1.5 mL) was added a solution of 1-(3-chloropropyl)-1-(4-fluorophenyl)-1,3-dihydroisobenzofuran-5-carbonitrile (9) (473 mg, 1.5 mmol) in acetonitrile (1.5 mL) and diisopropylethylamine (280 mL, 1.6 mmol). After heating of the resulting mixture at 75 °C under stirring for 16 h, the resin was filtered off. The resin was subsequently washed with acetonitrile (3 X 2.5 mL), methanol (3 X 2.5 mL), and dichloromethane (3 X 2.5 mL). The resin was suspended in N,N-dimethylformamide and diisopropylethylamine (280 mL, 1.6 mmol) and acetic anhydride (140 mL, 1.5 mmol) was added. After stirring of the resulting mixture for 16 h the resin was filtered off and washed with N,N-dimethylformamide (3 X 2.5 mL), methanol (3 X 2.5 mL), and dichloromethane (3 X 2.5 mL).
The intermediate resin was suspended in N,N-dimethylformamide (2 mL) and a solution of iodomethan (187 mL, 3.0 mmol) in N,N-dimethylformamide was added. After stirring of the resulting mixture for 16 h at room temperature, the resin was filtered off and washed with N,N-dimethylformamide (3 X 2.5 mL), methanol (3 X 2.5 mL), and dichloromethane (3 X 2.5 mL). To the resulting resin was added N,N-dimethylformamide (3.0 mL) and Diisopropylethylamine (165 mL, 0.9⁴ mmol) and the mixture was stirred for 16 h. The resin was filtered off and washed with methanol (2 X 2.0 mL). The cleavage solution and the washing solutions were collected and the solvent evaporated *in vacuo*. The remaining oil was purified by ion exchange chromatography using an 6 mL Varian SCX column (1225-6011). The column was preconditioned with 10% acetic acid in methanol (3 mL) and the crude product was loaded on the column in a 2:1 mixture of methanol and 1-methyl-2-pyrrolidinone (3 mL). After the column was washed with methanol (18 mL) and acetonitrile (3 mL) the product was eluted from the column with 4 N ammonia in methanol (4 mL) and subsequent evaporation of the solvents *in vacuo* afforded 13.9 mg (10%) of the title compound as an oil: LC/MS (m/z) 454 (MH⁺), Rt= 6.13 , purity: 98 %. The following compounds were prepared analogously:
1-(4-Fluorophenyl)-1-[3-[[2-(3-methoxyphenyl)ethyl]methylamino]propyl]-1,3-dihydroisobenzofuran-5-carbonitrile (**4b**): LC/MS (m/z) 445 (MH⁺), Rₜ = 8.58, purity: 88%
1-(4-Fluorophenyl)-1-[3-[[2-(3-methoxyphenyl)ethyl](prop-2-en-1-yl)amino]propyl]-1,3-dihydroisobenzofuran-5-carbonitrile (**4c**): ¹H NMR (CDCl₃) δ 1.30-1.60 (m, 2H), 2.00-2.20 (m, 2H), 2.40-2.55 (m, 2H), 2.55-2.70 (m, 4H), 3.00-3.15 (broad s, 2H), 3.80 (s, 3H), 5.05-5.20 (m, 4H), 5.75-5.85 (m, 1H), 6.65-6.80 (m, 3H), 7.00 (t, 2H), 7.20 (t, 1H), 7.30 (d, 1H), 7.40 (m, 2H), 7.50 (s, 1H), 7.60 (d, 1H); LC/MS (m/z) 471 (MH⁺), Rₜ = 8.85 , purity: 91%
1-(4-Fluorophenyl)-1-[3-[[2-(2-methoxyphenyl)ethyl](prop-2-en-1-yl)amino]propyl]-1,3-dihydroisobenzofuran-5-carbonitrile (**4d**): ¹H NMR (CDCl₃) δ 1.25-1.40 (m, 1H), 1.40-1.55 (m, 1H), 2.05-2.25 (m, 2H), 2.40-2.50 (m, 2H), 2.50-2.65 (m, 2H), 2.65-2.75 (m, 2H), 3.00-3.15 (m, 2 H), 3.80 (s, 3H); 5.05-5.20 (m, 4H), 5.75-5.90 (m, 1H), 6.75-6.90 (m, 2H), 6.95-7.10 (m, 3H), 7.20 (t, 1H), 7.30 (d, 1H), 7.35-7.45 (m, 2H), 7.45 (s, 1H), 7.60 (d, 1H); LC/MS (m/z) 471 (MH⁺), Rₜ = 7.82 , purity: >89%
1-[3-[[2-(2,5-Dimethoxyphenyl)ethyl]methylamino]propyl]-1-(4-fluorophenyl)-1,3-dihydroisobenzofuran-5-carbonitrile (**4e**): LC/MS (m/z) 475 (MH⁺), Rₜ = 8.68, purity: 94%
1-[3-[[2-(2,5-Dimethoxyphenyl)ethyl](prop-2-en-1-yl)amino]propyl]-1-(4-fluorophenyl)-1,3-dihydroisobenzofuran-5-carbonitrile (**4f**): LC/MS (m/z) 500 (MH⁺), Rₜ = 8.95 , purity: 90%
1-(4-Fluorophenyl)-1-[3-[[2-phenoxyethyl]methylamino]propyl]-1,3-dihydroisobenzofuran-5-carbonitrile (**4g**): LC/MS (m/z) 431 (MH⁺), Rₜ = 8.58 , purity: 95%
1-[3-[[2-(1*H*-Indolyl-3-yl)ethyl](prop-2-en-1-yl)amino]propyl]-1-(4-fluorophenyl)-1,3-dihydroisobenzofuran-5-carbonitrile (**4h**): LC/MS (m/z) 480 (MH⁺), Rₜ = 8.87 , purity: 93%
1-(4-Fluorophenyl)-1-[3-[[2-phenoxyethyl](prop-2-en-1-yl)amino]propyl]-1,3-dihydroisobenzofuran-5-carbonitrile (**4i**): LC/MS (m/z) 457 (MH⁺), Rₜ = 6.40 , purity:>99%
1-(4-Fluorophenyl)-1-[3-[[3-(2-methoxyphenyl)propyl]methylamino]propyl]-1,3-dihydroisobenzofuran-5-carbonitrile (**4j**): LC/MS (m/z) 459 (MH⁺), Rₜ = 6.43 , purity:>99%
1-(4-Fluorophenyl)-1-[3-[[3-(2-methoxyphenyl)propyl](prop-2-en-1-yl)amino]propyl)-1,3-dihydroisobenzofuran-5-carbonitrile (**4k**): LC/MS (m/z) 485 (MH⁺), Rₜ = 6.77 , purity: >99%
1-(4-Fluorophenyl)-1-[3-[[3-(3-methoxyphenyl)propyl](prop-2-en-1-yl)amino]propyl]-1,3-dihydroisobenzofuran-5-carbonitrile (**4l**): LC/MS (m/z) 485 (MH⁺), Rₜ = 6.63 , purity: >99%
1-(4-Fluorophenyl)-1-[3-[[3-(2-methoxyphenoxy)propyl]methylamino]propyl]-1,3-dihydroisobenzofuran-5-carbonitrile (**4m**): LC/MS (m/z) 475 (MH⁺), Rₜ = 6.20 , purity: >99%
1-(4-Fluorophenyl)-1-[3-[[3-(2-methoxyphenoxy)propyl](prop-2-en-1-yl)amino]propyl]-1,3-dihydroisobenzofuran-5-carbonitrile (**4n**): LC/MS (m/z) 501 (MH⁺), Rₜ = 6.50 , purity: >99%
1-(4-Fluorophenyl)-1-[3-[[3-(3-methoxyphenoxy)propyl]methylamino]propyl]-1,3-dihydroisobenzofuran-5-carbonitrile (**4o**): LC/MS (m/z) 475 (MH⁺), Rₜ = 6.35 , purity: >99%
1-(4-Fluorophenyl)-1-[3-[[3-(3-methoxyphenoxy)propyl](prop-2-en-1-yl)amino]propyl]-1,3-dihydroisobenzofuran-5-carbonitrile (**4p**): LC/MS (m/z) 501 (MH⁺), Rₜ = 6.65 , purity: >99%
1-[3-[(2-Benzyloxyethyl)methylamino]propyl]-1-(4-fluorophenyl)-1,3-dihydroisobenzofuran-5-carbonitrile (**4q**): LC/MS (m/z) 445 (MH⁺), Rₜ = 6.18 , purity: 98%
1-[3-[(2-Benzyloxyethyl)(prop-2-en-1-yl)amino]propyl]-1-(4-fluorophenyl)-1,3-dihydroisobenzofuran-5-carbonitrile (**4r**): LC/MS (m/z) 471 (MH⁺), Rₜ = 6.55 , purity: 97%
1-[3-[[3-(1*H*-Indol-3-yl)propyl]methylamino]propyl]-1-(4-fluorophenyl)-1,3-dihydroisobenzofuran-5-carbonitrile (**4s**): LC/MS (m/z) 468 (MH⁺), Rₜ= 6.28 , purity:80%
1-[3-[[3-(1*H*-Indol-3-yl)propyl](prop-2-en-1-yl)amino]propyl]-1-(4-fluorophenyl)-1,3-dihydroisobenzofuran-5-carbonitrile (**4t**): LC/MS (m/z) 494 (MH⁺), Rₜ= 6.60 , purity:82%
1-[3-[[3-(1*H*-Indol-3-yl)propyl](prop-2-yn-1-yl)amino]propyl]-1-(4-fluorophenyl)-1,3-dihydroisobenzofuran-5-carbonitrile (**4u**): LC/MS (m/z) 492 (MH⁺), Rₜ = 6.59 , purity:73%

### Example 5

5-hydroxymethyl-1,4-benzodioxan (**5**). To a suspension of lithium aluminum hydride (7.0 g, 0.18 mol) in dry diethyl ether (100 mL) was added a solution of ethyl 1,4-benzodioxan-5-carboxylate (35 g, 0.17 mol) in diethyl ether (100 mL). After boiling under reflux for 2 h, the reaction mixture was cooled to 0 °C and carefully treated with water (35 mL) and 4 N aqueous sodium hydroxide (35 mL). The resulting mixture was filtered and dried (Na₂SO₄). Evaporation of the solvents afforded 25 g (88%) crystalline title compound: mp 51-53 °C; ¹H NMR (CDCl₃) δ 2.50 (s, 1H), 4.20-4.3 ( m, 4H), 4.60 (s, 2H), 6.75-6.90 (m, 3H).

### Example 6

2-(1,4-benzodioxan-5-yl)acetic acid (**6**). To a solution of 5-hydroxymethyl-1,4-benzodioxan (8.0 g, 48 mmol) in dichloromethane (200 mL) was added two droplets of N,N-dimethylformamide and thionyl chloride (5.0 mL, 68 mmol) at room temperature. After the resulting solution was boiled under reflux for 1 h and subsequently cooled to room temperature water (100 mL) was added. The phases were separated and the organic phase was dried (MgSO₄) and the solvents evaporated *in vacuo*. A solution of the remaining oil (8.5 g, 46 mmol) was added to a mixture of sodium cyanide (5.0 g, 102 mmol) and N,N-dimethylformamide (100 mL) at room temperature. After stirring for 16 h at room temperature ice was added and the resulting slurry was extracted with diethyl ether (2 X 250 mL). The collected organic phases were washed with saturated calcium chloride, dried (Na₂SO₄) and the solvents were evaporated *in vacuo*. A mixture of the remaining oil (6.0 g, 34 mmol), ethanol (200 mL), sodium hydroxide (6.0 g) and water (6 mL) was boiled under reflux for 16 h. After evaporation of the solvents *in vacuo*, water (200 mL) was added and the resulting slurry was extracted with diethyl ether (2 X 200 mL). The collected organic phases were washed with brine, dried (Na₂SO₄) and the solvents were evaporated *in vacuo* affording 4.0 g (43%) of the title compound as an oil: ¹H NMR (CDCl₃) δ 3.65 (s, 2H), 4.15-4.30 (m, 4H), 6.70-6.85 (m, 3H).

### Example 7

5-(2-bromoethyl)-1,4-benzodioxan (**7a**). To a solution of 2-(1,4-benzodioxan-5-yl)acetic acid (**6**) (4.0 g, 21 mmol) in tetrahydrofuran (200 mL) was added lithium aluminum hydride (1.0 g, 26 mmol). After boiling under reflux for 2 h the reaction mixture was cooled to 0 °C and carefully treated with water (1 mL) and 4 N aqueous sodium hydroxide (1 mL). The resulting mixture was filtered and dried (Na₂SO₄). Evaporation of the solvents afforded crude intermediate alcohol (3.9 g, 21 mmol) as an oil which was used without further purification. To a solution of the intermediate alcohol and tetrabromomethane (8.8 g, 27 mmol) in acetonitrile (120 mL) was added triphenylphosphine (6.3 g, 24.9 mmol) in small portions at 0 °C. After reaction for further 15 minutes at 0 °C the solvents were evaporated *in vacuo* and the remaining oil was purified by column chromatography (ethyl acetate/heptane 66:34) affording 5.5 g (99%) of the title compound as an oil: ¹H NMR (CDCl₃) δ 3.15 (t, 2H), 3.55 (t, 2H), 4.20-4.35 (m, 4H), 6.65-6.85 (m, 3H).

The following compounds were prepared analogously:
5-(3-Bromopropyl)-1,4-benzodioxan (**7b**): (oil) ¹H NMR (CDCl₃) δ 2.15 (qui, 2H), 2.75 (t, 2H), 3.40 (t, 2H), 4.20-4.30 (m, 4H), 6.65-6.75 (m, 3H).
5-(4-Bromobutyl)-1,4-benzodioxan (**7c**): (oil) ¹H NMR (CDCl₃) δ 1.70-1.80 (qui, 2H), 1.85-1.90 (qui, 2H), 2.60 (t, 2H), 3.40 (t, 2H), 4.25 (m, 4H), 6.65-6.75 (m, 3H).
1-(2-Bromoethoxy)-2-methoxybenzene (**7d**): (oil) ¹H NMR (CDCl₃) δ 3.65 (t, 2H), 3.85 (s, 3H), 4.30 (t, 2H), 6.80-7.05 (m, 4H).
1-(2-Bromoethoxy)-3-methoxybenzene (**7e**): (oil) ¹H NMR (CDCl₃) δ 3.60 (t, 2H), 3.80 (s, 3H), 4.25 (t, 2H), 6.45-6.55 (m, 3H), 7.15 (t, 1H).

### Example 8

4-(1,4-Benzodioxan-5-yl)butanoic acid (**8a**). Neat 5-(4-bromoethyl)-1,4-benzodioxan (7c) (18.0 g, 74 mmol) was added to a mixture of diethyl malonate (12 g, 75 mmol), potassium tert-butoxide (8.4 g, 75 mmol), toluene (250 mL) and dimethyl sulfoxide (50 mL) at room temperature. The resulting mixture was heated at 50 °C for 3 h, cooled to room temperature and water was added. After the slurry was acidified with concentrated hydrochloric acid the phases were separated. The organic phase was dried (Na₂SO₄) and the solvents evaporated *in vacuo*. The remaining oil was dissolved in ethanol (200 mL) and 9 N aqueous sodium hydroxide. After boiling of the resulting mixture under reflux for 15 minutes the solution was stirred at room temperature for 1 h. The solvents were evaporated and the remaining oil was diluted in water (200 mL) and extracted with diethyl ether (2 X 100 mL). The aqueous phase was acidified with 4 N hydrochloric acid and extracted with ethyl acetate (2 X 200 mL). Drying of the collected organic phases and evaporation of the solvents *in vacuo* afforded the intermediate dicarboxylic acid as an oil (5.0 g). The crude oil was diluted in pyridine (10 mL) and the resulting solution was heated at 115. °C for 1 h. After cooling to room temperature, water (50 mL) was added and the aqueous phase was acidified with 4 N hydrochloric acid. The resulting slurry was extracted with diethyl ether (2 X 50 mL) and the collected organic phases were dried (Na₂SO₄). Evaporation of the solvents *in vacuo* afforded 3.8 g (23%) of the title compound as an oil.

The following compound was prepared analogously:
3-(1,4-Benzodioxan-5-yl)propionic acid (**8b**): (oil) ¹H NMR (CDCl₃) δ 2.65 (t, 2H), 2.95 (t, 2H), 4.20-4.30 (m, 4H), 6:65-6.80 (m, 3H).

### Example 9

1-(3-Chloropropyl)-1-(4-fluorophenyl)-1,3-dihydroisobenzofuran-5-carbonitrile (9). To a mixture of 1-[3-(methylamino)propyl]-1-(4-fluorophenyl)-1,3-dihydroisobenzofuran-5-carbonitrile (43 g, 138 mmol), potassium carbonate (30 g, 217 mmol), and ethanol (400 mL) was added ethyl bromoacetate (20 mL, 180 mmol) at room temperature and the resulting mixture was boiled under reflux for 90 minutes. After cooling to room temperature water (800 mL) and ethyl acetate (500 mL) was added and the phases were separated. The organic phase was washed with brine, dried (Na₂SO₄) and the solvents evaporated *in vacuo*. The remaining oil (36 g, 101 mmol) was added slowly to a mixture of ethyl chloroformate (50 mL, 523 mmol), potassium carbonate (36 g, 260) and toluene (300 mL) at 90 °C. After boiling of the resulting mixture under reflux for 1 h and cooling to room temperature, the solvents were evaporated *in vacuo*. The remaining oil was purified by column chromatography (ethyl acetate/heptane 1:3) giving 15 g (34%) of the title compound as an oil: ¹H NMR (CDCl₃) δ 1.60-1.90 (m, 2H), 2.20-2.45 (m, 2H), 3.45-3.55 (m, 2H), 5.20 (m, 2H), 6.95-7.10 (t, 2H), 7.40-7.55 (m, 4H), 7.60 (d, 1H).

### Example 10

[2-(2-Methoxyphenoxy)ethyl]methylamine (**10a**). A solution of 1-(2-bromoethoxy)-2-methoxybenzene (**7d**) (7.7 g, 33 mmol) in a 33% solution of methylamine in ethanol was heated at 80 °C in a sealed tube for 16 h. After cooling to room temperature, the solvents were evaporated *in vacuo*. A 2 N aqueous solution of sodium hydroxide was added to the remaining oil and the resulting slurry was extracted with ethyl acetate (2 X 250 mL). The collected organic phases were dried (Na₂SO₄) and the solvents evaporated in *vacuo* giving 5.9 g (98%) of the title compound as an oil: ¹H NMR (CDCl₃) δ 1.85 (broad s, 1H), 2.50 (s, 3H), 3.00 (t, 2H), 3.85 (s, 3H), 4.10 (t, 2H), 6.85-6.95 (m, 4H).

The following compound was prepared analogously:
[2-(3-Methoxyphenoxy)ethyl]methylamine (**10b**): (oil) ¹H NMR (CDCl₃) δ 1.85 (broad s, 1H), 2.50 (s, 3H), 2.95 (t, 2H), 3.80 (s, 3H), 4.05 (t, 2H), 6.45-6.55 (m, 3H), 7.15 (t, 1H).

### Example 11

2-(4-Chlorobutyl)-dioxolan-4-ylmethoxymethyl polystyrene (**11a**). A 2 L round bottom flask was charged with 2,2-dimethyldioxolan-4-ylmethoxymethyl polystyrene (90 g, 72 mmol, commercially available as (±)-1-(2,3-isopropylidene) glycerol polystyrene from Calbiochem-Novabiochem, cat. no. 01-64-0291). Toluene (900 mL) followed by p-toluenesulfonic acid mono hydrate (5.0 g, 26 mmol), sodium sulfate (25 g), and readily available 5-chloropentanal (25.5 g, 211 mmol) were added and the mixture heated at reflux for 12 h. The reflux condenser was replaced by a Dean-Stark apparatus and the mixture was heated at reflux for an additional 3 h. After cooling of the reaction mixture to 60 °C, the resin was filtered and washed with toluene (200 mL), tetrahydrofuran/pyridine (1:1, 200 mL), tetrahydrofuran/water/pyridine (10:10:1, 200 mL), methanol (200 mL), water (200 mL), tetrahydrofuran (200 mL), dichloromethane (200 mL), methanol (3 X 200 mL), and dichloromethane (3 X 200 mL). The resin was dried *in vacuo* (55 °C, 12 h) to yield the title compound **11 a** (97 g).

The following compounds were prepared analogously:
2-(3-Chloropropyl)-dioxolan-4-ylmethoxymethyl polystyrene (**11b**)
2-(5-Chloropentyl)-dioxolan-4-ylmethoxymethyl polystyrene (**11c**)

### Example 12

1-(3-[[3-(1*H*-Indol-3-yl)propyl]methylamino]propyl]-1-(4-fluorophenyl)-1,3-dihydroisobenzofuran-5-carbonitrile (**4s**). 2-(4-Chlorobutyl)-dioxolan-4-ylmethoxymethyl polystyrene (**11a**) (8.0 g, 6.1 mmol) was suspended in dry N,N-dimethylformamide (90 mL). Sodium iodide (3.38 g, 22.5 mmol) was added followed by diisopropylethylamine (6.30 mL, 36 mmol) and 1-[3-(methylaminp)propyl]-1-(4-fluorophenyl)-1,3-dihydroisobenzofuran-5-carbonitrile (5.56 g, 18 mmol). The reaction mixture was heated at 80 °C under stirring for 12 h. After cooling to room temperature, the resin was filtered and washed with with N,N-dimethylformamide (3 X 65 mL), methanol (3 X 60 mL), tetrahydrofuran (3 X 60 mL), and then subsequently with methanol and tetrahydrofuran (each approximately 40 mL, 5 cycles). Finally, the resin was washed with tetrahydrofuran (4 X 40 mL) and dried *in vacuo* (55 °C, 12 h, 9.5 g).
An aliquot of this material (147 mg, 0.112 mmol) and phenylhydrazine hydrochloride (43 mg, 0.297 mmol) were mixed in a reactor tube. A 0.5 M solution of anhydrous zinc chloride in acetic acid (1.5 mL) was added and the reaction tube was sealed. The reaction mixture was stirred for 12 h at 75 °C. After cooling to room temperature, the reaction mixture was filtered and the residual resin washed with dimethylsulfoxide (1.5 mL). To the combined filtrates was added saturated aqueous sodium bicarbonate solution (1.5 mL). The solution was loaded on a reversed solid phase extraction column (C-18, 1 g, Varian Mega Bond Elut®, Chrompack cat. no. 220508), pre-conditioned with methanol (3 mL) and water (3 mL). The column was washed with water (4 mL) and the product was eluted with methanol (4.5 mL). The resulting solution was loaded on an ion exchange column (SCX, 1 g, Varian Mega Bond Elut®, Chrompack cat. no. 220776), pre-conditioned with 10 % solution of acetic acid in methanol (3 mL) and the column was washed with methanol (4 mL) and acetonitrile (4 mL), followed by elution with 4 N solution of ammonia in methanol (4.5 mL). Evaporation of the volatile solvents afforded the title compound (**4s**) as a colourless oil (22 mg, 42 %). LC/MS (m/z) 468 (MH⁺), Rt = 4.30, purity: 83 %.

The following compounds were prepared analogously:
1-[3-[[2-(5-Methyl-1*H*-indol-3-yl)ethyl]methylamino]propyl]-1-(4-fluorophenyl)-1,3-dihydroisobenzofuran-5-carbonitrile (**12a**): LC/MS (m/z) 468 (MH⁺), Rt = 4.22, purity: 96 %.
1-[3-[[2-(7-Fluoro-1*H*-indol-3-yl)ethyl]methylamino]propyl]-1-(4-fluorophenyl)-1,3-dihydroisobenzofuran-5-carbonitrile (**12b**): ¹H NMR (CDCl₃) δ 1.2-1.4 (m, 1H), 1.4-1.55 (m,1H), 2.0-2.25 (m, 2H), 2.25 (s, 3H), 2.39 (t, 2H), 2.60 (t, 2H), 2.86 (t, 2H), 5.05-5.21 (m, 2H), 6.93-7.07 (m, 4H), 7.17-7.3 (m, 2H), 7.3-7.4 (m, 3H), 7.4-7.5 (m, 1H), 7.5-7.6 (m, 1H); LC/MS (m/z) 472 (MH⁺), Rt = 4.12, purity: 86 %.
5-Fluoro-1-[3-[[3-(5-methyl-1*H*-indol-3-yl)propyl]methylamino]propyl]-1-(4-fluorophenyl)-1,3-dihydroisobenzofuran (**12c**): LC/MS (m/z) 475 (MH⁺), Rt = 4.57, purity: 92 %.
5-Fluoro-1-[3-[[3-(7-fluoro-1*H*-indol-3-yl)propyl]methylamino]propyl]-1-(4-fluorophenyl)-1,3-dihydroisobenzofuran (**12d**): LC/MS (m/z) 479 (MH⁺), Rt = 4.47, purity: 94 %.
1-[3-[[3-(5-Methyl-1*H*-indol-3-yl)propyl]methylamino]propyl]-1-(4-fluorophenyl)-1,3-dihydroisobenzofuran-5-carbonitrile (**12e**): LC/MS (m/z) 482 (MH⁺), Rt = 4.54, purity: 80 %.
1-[3-[Ethyl[3-(1*H*-indol-3-yl)propyl]amino]propyl]-1-(4-fluorophenyl)-1,3-dihydroisobenzofuran-5-carbonitrile (**12f**): LC/MS (m/z) 482 (MH⁺), Rt = 4.31, purity: 94 %.
1-[3-[Ethyl[2-(5-methyl-1*H*-indol-3-yl)ethyl]amino]propyl]-1-(4-fluorophenyl)-1,3-dihydroisobenzofuran-5-carbonitrile (**12g**): LC/MS (m/z) 482 (MH⁺), Rt = 4.38, purity: 89 %.
1-[3-[[3-(7-Fluoro-1*H*-indol-3-yl)propyl]methylamino]propyl]-1-(4-fluorophenyl)-1,3-dihydroisobenzofuran-5-carbonitrile (**12h**): LC/MS (m/z) 486 (MH⁺), Rt = 4.16, purity: 79 %.
1-[3-[[3-(5-Fluoro-1*H*-indol-3-yl)propyl]methylamino]propyl]-1-(4-fluorophenyl)-1,3-dihydroisobenzofuran-5-carbonitrile (**12i**): ¹H NMR (CDCl₃) δ 1.23-1.39 (m, 1H), 1.39-1.54 (m, 1H), 1.80 (tt, 2H), 2.06-2.24 (m, 5H), 2.30 (t, 2H), 2.34 (t, 2H), 2.68 (t, 2H), 5.13 (d, 1H), 5.17 (d, 1H), 6.93 (dt, 2H), 6.99 (t, 2H), 7.21 (dd, 1H), 7.23-7.29 (m, 1H), 7.33 (d, 1H), 7.40 (dd, 2H), 7.47 (s, 1H), 7.55 (d, 1H), 8.01 (s, 1H); LC/MS (m/z) 486 (MH⁺), Rt = 4.12, purity: 98 %.
1-[3-[Ethyl[2-(5-fluoro-1*H*-indol-3-yl)ethyl]amino]propyl]-1-(4-fluorophenyl)-1,3-dihydroisobenzofuran-5-carbonitrile (**12j**): ¹H NMR (CDCl₃) δ 1.02 (t, 3H), 1.25-1.38 (m, 1H), 1.42-1.54 (m, 1H), 2.10 (ddd, 1H), 2.18 (ddd, 1H), 2.49 (t, 2H), 2.56 (q, 2H), 2.61-2.70 (m, 2H), 2.74-2.82 (m, 2H), 5.13 (d, 1H), 5.18 (d, 1H), 6.94 (dt, 2H), 6.99 (t, 2H), 7.19 (dd, 1H), 7.23-7.30 (m, 2H), 7.38 (dd, 2H), 7.47 (s, 1H), 7.54 (d, 1H), 8.01 (s, 1H); LC/MS (m/z) 486 (MH⁺), Rt = 4.24, purity: 95 %.
1-[3-[Ethyl[2-(7-fluoro-1*H*-indol-3-yl)ethyl]amino]propyl]-1-(4-fluorophenyl)-1,3-dihydroisobenzofuran-5-carbonitrile (**12k**): ¹H NMR (CDCl₃) δ 1.02 (t, 3H), 1.22-1.37 (m, 1H), 1.42-1.53 (m,1H), 2.0-2.2 (m, 2H), 2.36-2.6 (m, 4H), 2.67 (t, 2H), 2.81 (t, 2H), 5.12 (dd, 1H), 5.16 (d, 1H), 6.86-7:06 (m, 4H), 7.2-7.4 (m, 5H), 7.46 (d, 1H), 7.54 (d, 1H); LC/MS (m/z) 486 (MH⁺), Rt = 4.26, purity: 91 %.
1-[3-[[2-(5-Chloro-1*H*-indol-3-yl)ethyl]methylamino]propyl]-1-(4-fluorophenyl)-1,3-dihydroisobenzofuran-5-carbonitrile (**12l**): LC/MS (m/z) 488 (MH⁺), Rt = 4.30, purity: 85 %.
1-[3-[[3-(5-Chloro-1*H*-indol-3-yl)propyl]methylamino]propyl]-5-fluoro-1-(4-fluorophenyl)-1,3-dihydroisobenzofuran (**12m**): LC/MS (m/z) 495 (MH⁺), Rt = 4.64, purity: 94 %.
1-[3-[[4-(5-Methyl-1*H*-indol-3-yl)butyl]methylamino]propyl]-1-(4-fluorophenyl)-1,3-dihydroisobenzofuran-5-carbonitrile (**12n**): LC/MS (m/z) 496 (MH⁺), Rt = 4.50, purity: 78 %.
1-[3-[Ethyl[3-(5-methyl-1*H*-indol-3-yl)propyl]amino]propyl]-1-(4-fluorophenyl)-1,3-dihydroisobenzofuran-5-carbonitrile (**12o**): LC/MS (m/z) 496 (MH⁺), Rt = 4.50, purity: 92 %.
1-[3-[Ethyl[3-(7-fluoro-1*H*-indol-3-yl)propyl]amino]propyl]-1-(4-fluorophenyl)-1,3-dihydroisobenzofuran-5-carbonitrile (**12p**): LC/MS (m/z) 500 (MH⁺), Rt = 4.39, purity: 91 %.
1-[3-[Ethyl[3-(5-fluoro-1*H*-indol-3-yl)propyl]amino]propyl]-1-(4-fluorophenyl)-1,3-dihydroisobenzofuran-5-carbonitrile (**12q**): ¹H NMR (CDCl₃) δ 0.95 (t, 3H), 1.21-1.36 (m, 1H), 1.36-1.50 (m, 1H), 1.77 (tt, 2H), 2.10 (ddd, 1H), 2,18 (ddd, 1H), 2.34-2.50 (m, 6H), 2.65 (t, 2H), 5.12 (d, 1H), 5.15 (d, 1H), 6.90-7.04 (m, 4H), 7.20 (dd, 1H), 7.25 (dd, 1H), 7.30 (d, 1H), 7.36 (m, 2H), 7.45 (s, 1H), 7.52 (d, 1H), 8.12 (s, 1H); LC/MS (m/z) 500 (MH⁺), Rt = 4.35, purity: 94 %.
1-[3-[[3-(5-Chloro-1*H*-indol-3-yl)propyl]methylamino]propyl]-1-(4-fluorophenyl)-1,3-dihydroisoberizofuran-5-carbonitrile (**12r**): LC/MS (m/z) 502 (MH⁺), Rt = 4.55, purity: 91 %.
1-[3-[[2-(7-Chloro-1*H*-indol-3-yl)ethyl]ethylamino]propyl]-1-(4-fluorophenyl)-1,3-dihydroisobenzofuran-5-carbonitrile (**12s**): LC/MS (m/z) 502 (MH⁺), Rt = 4.41, purity: 80 %.
1-[3-[[2-(5-Chloro-1*H*-indol-3-yl)ethyl]ethylamino]propyl]-1-(4-fluorophenyl)-1,3-dihydroisobenzofuran-5-carbonitrile (**12t**): LC/MS (m/z) 502 (MH⁺), Rt = 4.44, purity: 95 %.
1-[3-[[2-(5,7-Difluoro-1*H*-indol-3-yl)ethyl]ethylamino]propyl]-1-(4-fluorophenyl)-1,3-dihydroisobenzofuran-5-carbonitrile(**12u**): LC/MS (m/z) 504 (MH⁺), Rt = 4.35, purity: 92 %.
1-[3-[[4-(5-Fluoro-1*H*-indol-3-yl)butyl]ethylamino]propyl]-1-(4-fluorophenyl)-1,3-dihydroisobenzofuran-5-carbonitrile (**12v**): LC/MS (m/z) 514 (MH⁺), Rt = 4.50, purity: 91 %.
1-[3-[[4-(5-Chloro-1*H*-indol-3-yl)butyl]methylamino]propyl]-1-(4-fluorophenyl)-1,3-dihydroisobenzofuran-5-carbonitrile (**12w**): LC/MS (m/z) 516 (MH⁺), Rt = 4.59, purity: 90 %.
1-[3-[[3-(5-Chloro-1*H*-indol-3-yl)propyl]ethylamino]propyl]-1-(4-fluorophenyl)-1,3-dihydroisobenzofuran-5-carbonitrile (**12x**): LC/MS (m/z) 516 (MH⁺), Rt = 4.56, purity: 97 %.
1-[3-[[3-(5,7-Difluoro-1*H*-indol-3-yl)propyl]ethylamino]propyl]-1-(4-fluorophenyl)-1,3-dihydroisobenzofuran-5-carbonitrile (**12y**): LC/MS (m/z) 518 (MH⁺), Rt = 4.47, purity: 90 %.
1-[3-[[2-(5-Bromo-1*H*-indol-3-yl)ethyl]methylamino]propyl]-1-(4-fluorophenyl)-1,3-dihydroisobenzofuran-5-carbonitrile (**12z**): LC/MS (m/z) 532 (MH⁺), Rt = 4.46, purity: 87 %.
1-[3-[[3-(5-Bromo-1*H*-indol-3-yl)propyl]methylamino]propyl]-1-(4-fluorophenyl)-1,3-dihydroisobenzofuran-5-carbonitrile (**12aa**): LC/MS (m/z) 546 (MH⁺), Rt = 4.59, purity: 88 %.
1-[3-[[2-(5-Bromo-1*H*-indol-3-yl)ethyl]ethylamino]propyl]-1-(4-fluorophenyl)-1,3-dihydroisobenzofuran-5-carbonitrile (**12ab**): LC/MS (m/z) 546 (MH⁺), Rt = 4.50, purity: 90 %.
1-[3-[[4-(5-Bromo-1*H*-indol-3-yl)butyl]methylamino]propyl]-1-(4-fluorophenyl)-1,3-dihydroisobenzofuran-5-carbonitrile (**12ac**): LC/MS (m/z) 560 (MH⁺), Rt = 4.61, purity: 90 %.
1-[3-[[3-(5-Bromo-1*H*-indol-3-yl)propyl)ethylamino)propyl)-1-(4-fluorophenyl)-1,3-dihydroisobenzofuran-5-carbonitrile (**12ad**): LC/MS (m/z) 560 (MH⁺), Rt = 4.62, purity: 92 %.
1-[3-[Ethyl[2-(5-iodo-1*H*-indol-3-yl)ethyl]amino]propyl]-1-(4-fluorophenyl)-1,3-dihydroisobenzofuran-5-carbonitrile (**12ae**): LC/MS (m/z) 594 (MH⁺), Rt = 4.60, purity: 82 %.
1-[3-[Ethyl[3-(5-iodo-1*H*-indol-3-yl)propyl]amino]propyl]-1-(4-fluorophenyl)-1,3-dihydroisobenzofuran-5-carbonitrile (**12af**): LC/MS (m/z) 608 (MH⁺), Rt = 4.72, purity: 71 %.
1-[2-[[4-(5-Chloro-1*H*-indol-3-yl)butyl]methylamino]ethyl]-1-(4-fluorophenyl)-1,3-dihydroisobenzofuran-5-carbonitrile (**12ag**): LC/MS (m/z) 502 (MH⁺), Rt = 4.50, purity: 90 %.
1-[2-[[4-(5-Bromo-1*H*-indol-3-yl)butyl]methylamino]ethyl]-1-(4-fluorophenyl)-1,3-dihydroisobenzofuran-5-carbonitrile (**12ah**): LC/MS (m/z) 546 (MH⁺), Rt = 4.55, purity: 83 %.
1-[4-[[2-(5,7-Difluoro-1*H*-indol-3-yl)ethyl]methylamino]butyl]-1-(4-fluorophenyl)-1,3-dihydroisobenzofuran-5-carbonitrile (**12ai**): LC/MS (m/z) 504 (MH⁺), Rt = 4.36, purity: 87 %.
1-[4-[[2-(7-Chloro-1*H*-indol-3-yl)ethyl]methylamino]butyl]-1-(4-fluorophenyl)-1,3-dihydroisobenzofuran-5-carbonitrile (**12aj**): LC/MS (m/z) 502 (MH⁺), Rt = 4.42, purity: 70 %.
1-[4-[[2-(5-Chloro-1*H*-indol-3-yl)ethyl]methylamino]butyl]-1-(4-fluorophenyl)-1,3-dihydroisobenzofuran-5-carbonitrile (**12ak**): LC/MS (m/z) 502 (MH⁺), Rt = 4.45, purity: 91 %.
1-[4-[[2-(5-Bromo-1*H*-indol-3-yl)ethyl]methylamino]butyl]-1-(4-fluorophenyl)-1,3-dihydroisobenzofuran-5-carbonitrile (**12al**): LC/MS (m/z) 546 (MH⁺), Rt = 4.48, purity: 90 %.
1-[4-[[2-(5-Methyl-1*H*-indol-3-yl)ethyl]methylamino]butyl]-1-(4-fluorophenyl)-1,3-dihydroisobenzofuran-5-carbonitrile (**12am**): LC/MS (m/z) 482 (MH⁺), Rt = 4.37, purity: 87 %.
1-[4-[[2-(5-Iodo-1*H*-indol-3-yl)ethyl]methylamino]butyl]-1-(4-fluorophenyl)-1,3-dihydroisobenzofuran-5-carbonitrile (**12an**): LC/MS (m/z) 594 (MH⁺), Rt = 4.57, purity: 83 %.
1-[4-[[2-(5-(2-methyl-2-propyl)-1*H*-indol-3-yl)ethyl]methylamino]butyl]-1-(4-fluorophenyl)-1,3-dihydroisobenzofuran-5-carbonitrile (**12ao**): LC/MS (m/z) 524 (MH⁺), Rt = 4.85, purity: 91 %.
1-[4-[[2-(5-(2-Propyl)-1*H*-indol-3-yl)ethyl]methylamino]butyl]-1-(4-fluorophenyl)-1,3-dihydroisobenzofuran-5-carbonitrile (**12ap**): LC/MS (m/z) 510 (MH⁺), Rt = 4.72, purity: 92 %.

### Example 13

1-[3-[[2-(5-Methyl-1*H*-indol-3-yl)ethyl](prop-2-en-1-yl)amino]propyl]-1-(4-fluorophenyl)-1,3-dihydroisobenzofuran-5-carbonitrile (**13a**).
2-(3-Chloropropyl)-1,3-dioxolan-4-ylmethoxymethyl polystyrene (2.0 g, 1.6 mmol) was suspended in dry N,N-dimethylformamide (15 mL). Sodium iodide (0.67 g, 4.5 mmol) was added followed by diisopropylethylamine (1.70 mL, 9.6 mmol) and allyl amine (0.28 g, 4.8 mmol). The reaction mixture was heated at 80 °C under stirring for 12 h. After cooling to room temperature, the resin was filtered and washed with N,N-dimethylformamide (3 X 15 mL), methanol (3 X 15 mL), tetrahydrofuran (3 X 15 mL), and subsequently with methanol and tetrahydrofuran (each 10 mL, 5 cycles). Finally, the resin was washed with tetrahydrofuran (4 X 10 mL) and dried in *vacuo* (55 °C, 12 h). The resin was then suspended in dry N,N-dimethylformamide (20 mL). Sodium iodide (0.60 g, 4.0 mmol) was added followed by diisopropylethylamine (0.48 mL, 2.7 mmol) and 1-(3-chloropropyl)-1-(4-fluorophenyl)-1,3-dihydroisobenzofuran-5-carbonitrile (**9**) (0.79 g, 2.5 mmol). The reaction mixture was stirred for 12 h at 80 °C. After cooling to room temperature, the resin was filtered and washed with with N,N-dimethylformamide (3 X 15 mL), methanol (3 X 15 mL), tetrahydrofuran (3 X 15 mL), and then subsequently with methanol and tetrahydrofuran (each ca. 15 mL, 5 cycles). Finally, the resin was washed with tetrahydrofuran (4 X 15 mL) and dried *in vacuo* (55 °C, 12 h, 2.1 g).
An aliquot of this material (120 mg, ca. 0.08 mmol) and 4-methylphenylhydrazine hydrochloride (ca. 40 mg, 0.20 mmol) were mixed in a reactor tube. A 0.5 M solution of anhydrous zinc chloride in acetic acid (1.5 mL) was added and the reaction tube was sealed. The reaction mixture was stirred for 12 h at 75 °C. After cooling to room temperature, the reaction mixture was filtered and the residual resin washed with dimethylsulfoxide (1.5 mL). To the combined filtrates was added saturated aqueous sodium bicarbonate solution (1.5 mL). The solution was loaded on a reversed phase column (C-18, 1 g, Varian Mega Bond Elut®, Chrompack cat. no. 220508), pre-conditioned with methanol (3 mL) and water (3 mL). The column was washed with water (4 mL) and the product was eluted with methanol (4.5 mL). After evaporation of the volatile solvents, the crude product was purified by preparative reversed phase HPLC chromatography. The resulting solution was loaded on an ion exchange column (SCX, 1 g, Varian Mega Bond Elut®, Chrompack cat. no. 220776), pre-conditioned with 10 % solution of acetic acid in methanol (3 mL) and the column was washed with methanol (4 mL) and acetonitrile (4 mL), followed by elution with 4 N solution of ammonia in methanol (4.5 mL). Evaporation of the volatile solvents afforded the title compound (**13a**) as a colorless oil (2 mg, 4 µmol, 5 %). LC/MS (m/z) 494 (MH⁺), Rt = 4.44, purity: 93 %.

The following compounds were prepared analogously:
1-[3-[[2-(5-Fluoro-1*H*-indol-3-yl)ethyl](prop-2-en-1-yl)amino]propyl]-1-(4-fluorophenyl)-1,3-dihydroisobenzofuran-5-carbonitrile (**13b**): LC/MS (m/z) 498 (MH⁺), Rt = 4.31, purity: 96 %.
1-[3-[[2-(7-Fluoro-1*H*-indol-3-yl)ethyl](prop-2-en-1-yl)amino]propyl]-1-(4-fluorophenyl)-1,3-dihydroisobenzofuran-5-carbonitrile (**13c**): LC/MS (m/z) 498 (MH⁺), Rt = 4.34, purity: 86 %.
1-[3-[[3-(5-Fluoro-1*H*-indol-3-yl)propyl](prop-2-en-1-yl)amino]propyl]-1-(4-fluorophenyl)-1,3-dihydroisobenzofuran-5-carbonitrile (**13d**): LC/MS (m/z) 512 (MH⁺), Rt = 4.48, purity: 96 %.
1-[3-[[3-(7-Fluoro-1*H*-indol-3-yl)propyl](prop-2-en-1-yl)amino]propyl]-1-(4-fluorophenyl)-1,3-dihydroisobenzofuran-5-carbonitrile (**13e**): LC/MS (m/z) 512 (MH⁺), Rt = 4.49, purity: 78 %.
1-[3-[[2-(5-Chloro-1*H*-indol-3-yl)ethyl](prop-2-en-1-yl)amino]propyl]-1-(4-fluorophenyl)-1,3-dihydroisobenzofuran-5-carbonitrile (**13f**): LC/MS (m/z) 514 (MH⁺), Rt = 4.52, purity: 86 %.
1-[3-[[2-(5,7-Difluoro-1*H*-indol-3-yl)ethyl]propylamino]propyl]-1-(4-fluorophenyl)-1,3-dihydroisobenzofuran-5-carbonitrile (**13g**): LC/MS (m/z) 518 (MH⁺), Rt = 4.47, purity: 89 %.
1-[3-[[2-[5-(2-Propyl)-1*H*-indol-3-yl]ethyl](2-propyl)amino]propyl]-1-(4-fluorophenyl)-1,3-dihydroisobenzofuran-5-carbonitrile(**13h**): LC/MS (m/z) 524 (MH⁺), Rt = 4.78, purity: 96 %.
1-[3-[[3-(4-Fluoro-7-methyl-1*H*-indol-3-yl)propyl](prop-2-en-1-yl)amino]propyl]-1-(4-fluorophenyl)-1,3-dihydroisobenzofuran-5-carbonitrile(**13i**): LC/MS (m/z) 526 (MH⁺), Rt = 4.65, purity: 83 %.
1-[3-[[2-(4-Chloro-7-methyl-1*H*-indol-3-yl)ethyl](prop-2-en-1-yl)amino]propyl]-1-(4-fluorophenyl)-1,3-dihydroisobenzofuran-5-carbonitrile (**13j**): LC/MS (m/z) 528 (MH⁺), Rt = 4.67, purity: 79 %.
1-[3-[[3-(5-Chloro-1*H*-indol-3-yl)propyl](prop-2-en-1-yl)amino]propyl]-1-(4-fluorophenyl)-1,3-dihydroisobenzofuran-5-carbonitrile (**13k**): LC/MS (m/z) 528 (MH⁺), Rt = 4.63, purity: 78 %.
1-[3-[[2-(5-Pyrrolo[3,2-*h*]-1*H*-quinolin-3-yl)ethyl](prop-2-en-1-yl)amino]propyl]-1-(4-fluorophenyl)-1,3-dihydroisobenzofuran-5-carbonitrile (**13l**): LC/MS (m/z) 531 (MH⁺), Rt = 3.43, purity: 91 %.
1-[3-[[3-(7-Fluoro-1*H*-indol-3-yl)propyl](2-furylmethyl)amino]propyl]-1-(4-fluorophenyl)-1,3-dihydroisobenzofuran-5-carbonitrile (**13m**): LC/MS (m/z) 552 (MH⁺), Rt = 4.58, purity: 82 %.
1-[3-[[4-(7-Carboxy-1*H*-indol-3-yl)butyl](prop-2-en-1-yl)amino]propyl]-1-(4-fluorophenyl)-1,3-dihydroisobenzofuran-5-carbonitrile (**13n**): LC/MS (m/z) 552 (MH⁺), Rt = 4.17, purity: 69 %.
1-[3-[[2-[5-Bromo-1*H*-indol-3-yl]ethyl]-propylamino]propyl]-1-(4-fluorophenyl)-1,3-dihydroisobenzofuran-5-carbonitrile (**13o**): LC/MS (m/z) 560 (MH⁺), Rt = 4.62, purity: 96 %.
1-[3-[[3-(1*H*-Indol-3-yl)propyl](2-phenoxyethyl)amino]propyl]-1-(4-fluorophenyl)-1,3-dihydroisobenzofuran-5-carbonitrile (**13p**): LC/MS (m/z) 574 (MH⁺), Rt = 4.78, purity: 93 %.
1-[3-[[2-(5-Methyl-1*H*-indol-3-yl)ethyl](2-phenoxyethyl-)amino]propyl]-1-(4-fluorophenyl)-1,3-dihydroisobenzofuran-5-carbonitrile (**13q**): LC/MS (m/z) 574 (MH⁺), Rt = 4.82, purity: 93 %.
1-[3-[[2-(5-Fluoro-1*H*-indol-3-yl)ethyl](2-phenoxyethyl)amino]propyl]-1-(4-fluorophenyl)-1,3-dihydroisobenzofuran-5-carbonitrile (**13r**): LC/MS (m/z) 578 (MH⁺), Rt =4.71, purity: 95 %.
1-[3-[[3-(1*H*-Pyrrolo[3,2-*h*]quinolin-3-yl)propyl](2-furylmethyl)amino]propyl]-1-(4-fluorophenyl)-1,3-dihydroisobenzofuran-5-carbonitrile (**13s**): LC/MS (m/z) 585 (MH⁺), Rt = 3.60, purity: 90%.
1-[3-[[3-(5-Methyl-1*H*-indol-3-yl)propyl](2-phenoxyethyl)amino]propyl]-1-(4-fluorophenyl)-1,3-dihydroisobenzofuran-5-carbonitrile(**13t**): LC/MS (m/z) 588 (MH⁺), Rt = 4.96, purity: 82 %.
1-[3-[[3-(5-Fluoro-1*H*-indol-3-yl)propyl](2-phenoxyethyl)amino]propyl]-1-(4-fluorophenyl)-1,3-dihydroisobenzofuran-5-carbonitrile (**13u**): LC/MS (m/z) 592 (MH⁺), Rt = 4.82, purity: 90 %.
1-[3-[[2-(5,7-Difluoro-1*H*-indol-3-yl)ethyl](2-phenoxyethyl)amino]propyl]-1-(4-fluorophenyl)-1,3-dihydroisobenzofuran-5-carbonitrile (**13v**): LC/MS (m/z) 596 (MH⁺), Rt = 4.84, purity: 92 %.
1-[3-[[4-(1*H*-Pyrrolo[3,2-*h*]quinolin-3-yl)butyl](2-furylmethyl)amino]propyl]-1-(4-fluorophenyl)-1,3-dihydroisobenzofuran-5-carbonitrile (**13w**): LC/MS (m/z) 599 (MH⁺), Rt = 3.71, purity: 83 %.
1-[3-[(2-Phenoxyethyl)[2-[5-(2-propyl)-1*H*-indol-3-yl]ethyl]amino]propyl]-1-(4-fluorophenyl)-1,3-dihydroisobenzofuran-5-carbonitrile (**13x**): LC/MS (m/z) 602 (MH⁺), Rt = 5.24, purity: 78 %.
1-[3-[[2-(5-Bromo-1*H*-indol-3-yl)ethyl](2-phenoxyethyl)amino]propyl]-1-(4-fluorophenyl)-1,3-dihydroisobenzofuran-5-carbonitrile (**13y**): LC/MS (m/z) 638 (MH⁺), Rt = 4.98, purity: 91 %.

### Example 14

1-(3-Iodopropyl)-1-(4-fluorophenyl)-1,3-dihydroisobenzofuran-5-carbonitrile (**14a**). A solution/suspension of 1-(3-chloropropyl)-1-(4-fluorophenyl)-1,3-dihydroisobenzofuran-5-carbonitrile (20 g, 35 mmol, 80 % pure) and sodium iodide (285 g, 1.9 mol) in dry acetone (200 ml) was heated at reflux for 24 h. The mixture was evaporated, and partitioned between ether and water. The ether layer was separated, and was washed successively with water and brine. The organic extract was dried over anhydrous magnesium sulfate, filtered and evaporated to give 1-(3-iodopropyl)-1-(4-fluorophenyl)-1,3-dihydroisobenzofuran-5-carbonitrile (25,8 g, 99 %, 80 % pure) as a thick oil. ¹H NMR (CDCl₃) δ 1.6-1.9 (m, 2H), 2.21 (ddd, 1H), 2.31 (ddd, 1H), 3.16 (td, 2H), 5.12 (dt, 1H), 5.21 (dt, 1H), 7.02 (t, 2H), 7.41 (d, 2H), 7.43 (d, 1H), 7.51 (s, 1H), 7.62 (dq, 1H)

The following compounds were prepared analogously:
1-(2-Iodoethyl)-1-(4-fluorophenyl)-1,3-dihydroisobenzofuran-5-carbonitrile (**14b**): yellow oil, ¹H NMR (CDCl₃) δ 2.4-2.9 (m, 2H), 3.38 (dt, 1H), 3.46 (dt, 1H), 5.15 (d, 1H), 5.21 (d, 1H), 7.03 (t, 2H), 7.35-7.48 (m, 3H), 7.52 (s, 1H), 7.62 (d, 1H).
1-(4-lodobutyl)-1-(4-fluorophenyl)-1,3-dihydroisobenzofuran-5-carbonitrile (**14c**): yellow oil, ¹H NMR (CDCl₃) δ 1.1-1.5 (m, 2H), 1.81 (tt, 2H), 2.00-2.30 (m, 2H), 3.11 (t, 2H), 5.14 (d, 1H), 5.20 (d, 1H), 7.01 (t, 2H), 7.35-7.47 (m, 3H), 7.51 (s, 1H), 7.60 (d, 1H).

### Example 15

1-(3-(Ethylamino)propyl)-1-(4-fluorophenyl)-1,3-dihydroisobenzofuran-5-carbonitrile (**15a**). To a stirred solution of 1-(3-iodopropyl)-1-(4-fluorophenyl)-1,3-dihydroisobenzofuran-5-carbonitrile (12.9 g, 30 mmol, 8 % pure) in ethanol (150 mL) was added a solution of ethylamine (20.3 g, 450 mmol) in THF (50 mL) portionwise, and the mixture was stirred over night. The solution was evaporated, and was dissolved/suspended in water. The pH was adjusted to 12 using aqueous sodium hydroxide solution (2 M) and was extracted with ether. The organic extract was washed with brine, dried over anhydrous magnesium sulfate, filtered and evaporated to give an oil. This oil was purified by silica chromatography using 50% v/v ethyl acetate/heptane as eluent, followed by 10% v/v triethylamine/ 40% v/v ethyl acetate/heptane followed by 20% v/v triethylamine/ethyl acetate to give the title compound (5.52 g, 57%) as a pale yellow oil. ¹H NMR (CDCl₃) δ 1.05 (t, 3 H), 1.2-1.6 (m, 2H), 2.15 (ddd, 1H), 2.24 (ddd, 1H), 2.57 (q, 2H) 2.58 (t, 2H), 5.12 (dt, 1H), 5.20 (dt, 1H), 7.00 (t, 2H), 7.38 (d, 1H), 7.42 (dd, 2H), 7.49 (s, 1H), 7.58 (ddt, 1H).

The following compounds were prepared analogously:
1-(2-(Methylamino)ethyl)-1-(4-fluorophenyl)-1,3-dihydroisobenzofuran-5-carbonitrile (**15b**): yellow oil; ¹H NMR (CDCl₃) δ 2.38 (s, 3H), 2.33-2.72 (m, 4H), 5.13 (d, 1H), 5.20 (d, 1H), 7.01 (t, 2H), 7.37-7.47 (m, 3H), 7.50 (s, 1H), 7.59 (d, 1H).
1-(4-(Methylamino)butyl)-1-(4-fluorophenyl)-1,3-dihydroisobenzofuran-5-carbonitrile (**15c**): yellow oil; ¹H NMR (CDCl₃) δ 1.00-1.45 (m, 2H), 1.46 (tt, 2H), 2.10 (ddd, 1H), 2.21 (ddd, 1H), 2.37 (s, 3H), 2.50 (t, 2H), 5.13 (d, 1H), 5.19 (d, 1H), 7.00 (t, 2H), 7.34-7.46 (m, 3H), 7.49 (s, 1H), 7.59 (d, 1H).

### Pharmacological Testing

The affinity of the compounds of the invention to 5-HT_{1A} receptors was determined by measuring the inhibition of binding of a radioactive ligand at 5-HT_{1A} receptors as described in the following test:

### Inhibition of ³H-5-CT Binding to Human 5-HT_{1A} Receptors

By this method the inhibition by drugs of the binding of the 5-HT_{1A} agonist ³H-5-carboxamido tryptamine (³H-5-CT) to cloned human 5-HT_{1A} receptors stably expressed in transfected HeLa cells (HA7) (Fargin, *A. et al, J. Biol. Chem*., **1989,** *264,* 14848) is determined *in vitro*. The assay was performed as a modification of the method described by Harrington, M.A. *et al, J. Pharmacol. Exp. Ther*., **1994,** *268,* 1098. Human 5-HT_{1A} receptors (40 µg of cell homogenate) were incubated for 15 minutes at 37 °C in 50 mM Tris buffer at pH 7.7 in the presence of ³H-5-CT. Non-specific binding was determined by including 10 µM of metergoline. The reaction was terminated by rapid filtration through Unifilter GF/B filters on a Tomtec Cell Harvester. Filters were counted in a Packard Top Counter. The results obtained are presented in table 1 below.

The compounds of the invention have also been tested for their effect on re-uptake of serotonin in the following test:

### Inhibition of ³H-5-HT Uptake Into Rat Brain Synaptosomes

Using this method, the ability of drugs to inhibit the accumulation of ³H-5-HT into whole rat brain synaptosomes is determined *in vitro*. The assay was performed as described by Hyttel, J., *Psychopharmacology* **1978,** *60*, 13. The results obtained are presented in table 1:

**Table 1**

| **Compound No.** | **Inhibition of** ^{**3**}**H- 5-CT binding IC**_{**50**}**(nM) % inhibition at 100 nM** | **Inhibition of serotonin reuptake IC**_{**50**}**(nM) % inhibition at 100 nM** |
|---|---|---|
| **1a** | 39 | 60 |
| **1b** | 12 | 13 |
| **1c** | 53 | 85 |
| **2a** | 1.0 | 340 |
| **2b** | 6.4 | 40 |
| **2e** | 38 | 15 |
| **2f** | 8.6 | 14 |
| **2g** | 40 | 20 |
| **2j** | 41 | 9.7 |
| 2m | 4.7 | Not tested |
| **2n** | 15 | Not tested |
| **2o** | 12 | 31 |
| **4a** | 23 | 54 |
| **4b** | 63 | 59% inh. at 100 nM |
| **4c** | 11 | 4% inh. at 100 nM |
| **4d** | 4.5 | 7% inh. at 100 nM |
| **4e** | 17 | 160 |
| **4f** | 1.6 | 4% inh. at 100 nM |
| **4g** | 18 | 28% inh. at 100 nM |
| **4h** | 3.2 | 69 |
| **4i** | 1.9 | 26% inh. at 100 nM |
| **4j** | 6.1 | 78 |
| **4k** | 0.42 | 100 |
| **4l** | 76% inh. at 100 nM | 27% inh. at 100 nM |
| **4m** | 65% inh. at 100 nM | 74% inh. at 100 nM |
| **4n** | 14 | 39% inh. at 100 nM |
| **4o** | 26 | 73 |
| **4p** | 19 | 6% inh. at 100 nM |
| **4q** | 16 | 60% inh. at 100 nM |
| **4r** | 11 | 19% inh. at 100 nM |
| **4s** | 30 | 35 |
| **4t** | 69% inh. at 100 nM | 73% inh. at 100 nM |
| **4u** | 58% inh. at 100 nM | 44% inh. at 100 nM |
| **12b** | 43 | 10 |
| **12c** | 19 | 17 |
| **12d** | 31 | 12 |
| **12f** | 4.7 | 13 |
| **12i** | 27 | 20 |
| **12j** | 7.9 | 14 |
| **12k** | 3.6 | 8.4 |
| **12o** | 6.2 | 49% inh. at 100 nM |
| **12p** | 19 | 11 |
| **12q** | 12 | 6.3 |
| **12r** | 16 | 47% inh. at 100 nM |
| **12s** | 7.7 | 18 |
| **12u** | 9.0 | 22 |
| **12v** | 39 | 12 |
| **12x** | 14 | 50% inh. at 100 nM |
| **12aa** | 16 | 37% inh. at 100 nM |
| **12ab** | 20 | 50% inh. at 100 nM |
| **12ad** | 21 | 35% inh. at 100 nM |
| **12ae** | 11 | 49% inh. at 100 nM |
| **12af** | 31 | 38% inh. at 100 nM |
| **13b** | 7.4 | 44 |
| **13c** | 9.6 | 12 |
| **13d** | 15 | 21 |
| **13e** | 22 | 27 |
| **13f** | 31 | 16% inh. at 100 nM |
| **13g** | 18 | 49% inh. at 100 nM |
| **13j** | 16 | 61% inh. at 100 nM |
| **13k** | 19 | Not tested |
| **13p** | 23 | Not tested |
| **13q** | 12 | Not tested |
| **13r** | 8.9 | Not tested |
| **13t** | 23 | Not tested |
| **13u** | 22 | Not tested |
| **13v** | 23 | Not tested |
| **13x** | 26 | Not tested |
| **Pindolol*** | 100 | |
| **Paroxetine*** | - | 0.29 |

| Table 1 *reference compounds | | |
|---|---|---|
| | | |

Furthermore, the 5-HT_{1A} antagonistic activity of some of the compounds of the invention has been estimated in *vitro* at cloned 5-HT_{1A} receptors stably expressed in transfected HeLa cells (HA7). In this test, 5-HT_{1A} antagonistic activity is estimated by measuring the ability of the compounds to antagonize the 5-HT induced inhibition of forskolin induced cAMP accumulation. The assay was performed as a modification of the method described by Pauwels, P.J. *et al, Biochem. Pharmacol*. **1993**, *45*, 375.

As seen from the above, the compounds of the invention show affinity for the 5-HT_{1A} receptor. Furthermore, many of the compounds of the present invention possess valuable activity as serotonin re-uptake inhibitors.
Accordingly, the compounds are considered useful for the treatment of psychiatric and neurological disorders as mentioned previously.

### Pharmaceutical formulation

The pharmaceutical formulations of the invention may be prepared by conventional methods in the art. For example: Tablets may be prepared by mixing the active ingredient with ordinary adjuvants and/or diluents and subsequently compressing the mixture in a conventional tabletting machine. Examples of adjuvants or diluents comprise: corn starch, potato starch, talcum, magnesium stearate, gelatine, lactose, gums, and the like. Any other adjuvants or additives usually used for such purposes such as colourings, flavourings, preservatives etc. may be used provided that they are compatible with the active ingredients.
Solutions for injections may be prepared by dissolving the active ingredient and possible additives in a part of the solvent for injection, preferably sterile water, adjusting the solution to desired volume, sterilisation of the solution and filling in suitable ampules or vials. Any suitable additive conventionally used in the art may be added, such as tonicity agents, preservatives, antioxidants, etc.

The pharmaceutical compositions of this invention or those which are manufactured in accordance with this invention may be administered by any suitable route, for example orally in the form of tablets, capsules, powders, syrups, etc., or parenterally in the form of solutions for injection. For preparing such compositions, methods well known in the art may be used, and any pharmaceutically acceptable carriers, diluents, excipients, or other additives normally used in the art may be used.
Conveniently, the compounds of the invention are administered in unit dosage form containing said compounds in an amount of about 0.01 to 1000 mg. The total daily dose is usually in the range of about 0.05 - 500 mg, and most preferably about 0.1 to 50 mg of the active compound of the invention.

## Claims

1. An isobenzofuran having the general Formula I: 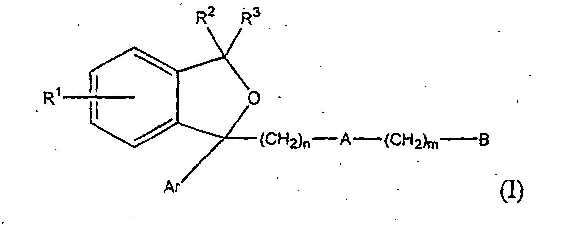 wherein
R¹ is hydrogen, halogen, triflubromethyl, trifluoromethylsulfonyloxy, C₁₋₆ alkyl, C₂₋₆ alkenyl, C₂₋₆ alkynyl, C₃₋₈ cycloalkyl, C₁₋₆ alkoxy, hydroxy, formyl, acyl, amino, C₁₋₆ alkylamino, C₂₋₁₂ dialkylamino, acylamino, C₁₋₆ alkoxycarbonylamino, aminocarbonylamino, C₁₋₆ alkylaminocarbonylamino, C₂₋₁₂ dialkylaminocarbonylamino, nitro, cyano, COOH, or COO-C₁₋₆ alkyl;
R² and R³ are each independently selected from hydrogen, trifluoromethyl, C₁₋₆ alkyl, C₂₋₆ alkenyl, C₂₋₆ alkynyl, C₃₋₈ cycloalkyl and C₁₋₆ alkoxy;
n is 1, 2, 3, 4 or 5;
m is 0 or 1; 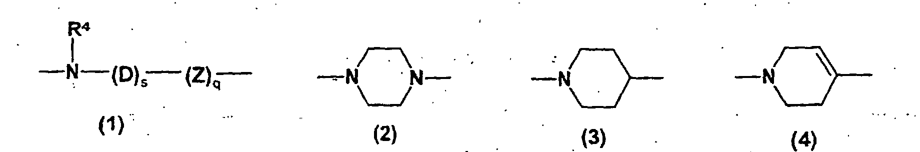 A is selected from the following groups:
wherein
Z is O or S;
s is 0 or 1;
R⁴ is hydrogen, C₁₋₆-alkyl, C₂₋₆-alkenyl, C₂₋₆-alkynyl, C₁₋₆-alkyl-Aryl, or C₁₋₆-alkyl-O-Aryl;
D is a spacer group selected from branched or straight chain C₁₋₆-alkylene, C₂₋₆-alkenylene and C₂₋₆-alkynylene;
B is a group selected from a group of formula (II), (III), and (IV) 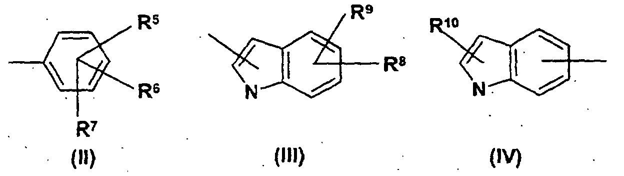 wherein R⁵, R⁶, R⁷, R⁸, R⁹ and R¹⁰ are each independently selected among the R¹ substituents;
or R⁸ and R⁹ together form a fused 5- or 6-membered ring optionally containing further heteroatoms; and the resulting heterocycle is optionally substituted with substituents selected among the R¹ substituents;
or two of the groups of R⁵, R⁶ and R⁷ are linked together thereby forming a ―O―(CH₂)ₚ―O― -bridge wherein p is 1 or 2;
Ar and Aryl are independently selected from the group consisting of phenyl, 2-thienyl, 3-thienyl, 2-furanyl, 3-furanyl, 2-pyrimidyl, 1-indolyl, 2-indolyl, 3-indolyl, 1-indol-2-onyl, 3-indol-2-onyl, 2- or 3-benzofuranyl, 2- or 3-benzothiophenyl, 1-naphthyl or 2-naphthyl, each optionally substituted with halogen, C₁₋₆ alkyl, C₁₋₆ alkoxy, C₁₋₆ alkylthio, hydroxy, C₁₋₆ alkylsulfonyl, cyano, trifluoromethyl, trifluoromethylsulfonyloxy, C₃₋₈ cycloalkyl, C₃₋₈ cycloalkyl-C₁₋₆ alkyl, nitro, amino, C₁₋₆ alkylamino, C₂₋₁₂ dialkylamino, acylamino or alkylenedioxy;
its enantiomers, and pharmaceutically acceptable acid addition salt thereof.

2. A compound of Claim 1, **characterised in that** R⁴ is methyl, ethyl, propyl; 2-propen-1-yl, 2-furylmethyl, 2-phenoxyethyl;

3. A compound of Claim 1, **characterised in that** at least one of R⁵, R⁶ and R⁷, is methoxy.

4. A compound of Claim 1, **characterised in that** Formula (II) is a benzodioxan group or a 1,2-methylenedioxybenzene group.

5. A compound of Claim 1, **characterised in that** Formula (III) is a 3-indolyl.

6. A compound of Claim 5, **characterised in that** the 3-indolyl is substituted in 5-position by methyl, fluoro, chloro, bromo, iodo, *t*-butyl or *i*-propyl, or in 7-position by fluoro, chloro or carboxy; or disubstituted by 5,7-difluoro, 4-fluoro-7-methyl or 4-chloro-7-methyl or the two substituents together form a pyridyl ring fused to the 3-indolyl.

7. A compound of Claim 1, **characterised in that** Formula (IV) is a 4-indolyl or a 5-indolyl group.

8. A compound of any of the Claims 1-7, **characterised in that** Ar is phenyl or phenyl substituted with halogen or CF₃;

9. A compound of any of the Claims 1 - 8, **characterised in that** R¹ is H, CN or F in the 5-position of the isobenzofuran group.

10. A compound of any of the Claims 1 - 9, **characterised in that** R² and R³ are selected from hydrogen or methyl.

11. A compound of any of the Claims 1 -10, **characterised in that** n = 2, 3 or 4.

12. A compound of any of the Claims 1 - 11, **characterised in that** m = 0.

13. The compound according to claim 1 which is
(-)-1-[3-[[4-(1,4-Benzodioxan-5-yl)butyl]methylamino]propyl]-1-(4-fluorophenyl)-1,3-dihydroisobenzofuran-5-carbonitrile,
1-[3-[[3-(1,4-Benzodioxan-5-yl)propyl]methylamino]propyl]-1-(4-fluorophenyl)-1,3-dihydroisobenzofuran-5-carbonitrile oxalate,
1-[3-[[2-(1,4-Benzodioxan-5-yl)ethyl]methylamino]propyl]-1-(4-fluorophenyl)-1,3-dihydroisobenzofuran-5-carbonitrile oxalate,
1-[3-[[1,4-Benzodioxan-5-ylmethyl]methylamino]propyl]-1-(4-fluorophenyl)-1,3-dihydroisobenzofuran-5-carbonitrile oxalate,
1-(4-Fluorophenyl)-1-[3-[4-(2-methoxyphenyl)piperazinyl]propyl]-1,3-dihydroisobenzofuran-5-carbonitrile,
1-(4-Fluorophenyl)-1-[3-[methyl[2-(2-methoxyphenoxy)ethyl]amino]propyl]-1,3-dihydroisobenzofuran-5-carbonitrile,
1-(4-Fluorophenyl)-1-[3-[methyl[2-(3-methoxyphenoxy)ethyl]amino]propyl]-1,3-dihydroisobenzofuran-5-carbonitrile,
(*S*)-1-[3-[[4-(1*H*-Indol-3-yl)butyl]methylamino]propyl]-1-(4-fluorophenyl)-1,3-dihydroisobenzofuran-5-carbonitrile,
1-[3-[[4-(1*H*-Indol-3-yl)butyl]methylamino]propyl]-1-phenyl-1,3-dihydroisobenzofuran,
(*S*)-1-[3-[[3-(1*H*-Indol-3-yl)propyl]methylamino]propyl]-1-(4-fluorophenyl)-1,3-dihydroisobenzofuran-5-carbonitrile,
1-[3-[[3-(1*H*-Indol-3-yl)propyl]methylamino]propyl]-1-phenyl-1,3-dihydroisobenzofuran,
5-[3-[[3-(1-Phenyl-1,3-dihydroisobenzofuran-1-yl)propyl]methylamino]propyl]-1,4-benzodioxane,
5-[3-[[3-[1-(3-Chlorophenyl)-1,3-dihydroisobenzofuran-1-yl]propyl]methylamino]propyl]-1,4-benzodioxane,
5-[3-[[3-[1-(4-Fluorophenyl)-1,3-dihydroisobenzofuran-1-yl]propyl]methylamino]propyl]-1,4-benzodioxane,
5-[3-[[3-[1-(3-Trifluoromethylphenyl)-1,3-dihydroisobenzofuran-1-yl]propyl]methylamino]propyl]-1,4-benzodioxane,
1-[3-[[3-(1,4-Benzodioxan-5-yl)propyl]methylamino]propyl]-1-(4-chlorophenyl)-1,3-dihydroisobenzofuran-5-carbonitrile,
1-[3-[4-(1*H*-Indol-4-yl)piperazinyl]propyl]-1-(4-fluorophenyl)-1,3-dihydroisobenzofuran-5-carbonitrile,
1-[3-[4-(1*H*-Indol-5-yl)piperazinyl]propyl]-1-(4-fluorophenyl)-1,3-dihydroisobenzofuran-5-carbonitrile,
1-[3-[4-(1*H*-Indol-3-yl)piperidinyl]propyl]-1-(4-fluorophenyl)-1,3-dihydroisobenzofuran-5-carbonitrile,
5-[3-[[3-[-5-Fluoro-1-(4-fluorophenyl)-1,3-dihydroisobenzofuran-1-yl]propyl]methylamino]propyl]-1,4-benzodioxane,
1-[3-[[2-(1*H*-Indolyl-3-yl)ethyl]methylamino]propyl]-1-(4-fluorophenyl)-1,3-dihydroisobenzofiiran-5-carbonitrile,
1-(4-Fluorophenyl)-1-[3-[[2-(3-methoxyphenyl)ethyl]methylamino]propyl]-1,3-dihydroisobenzofuran-5-carbonitrile,
1-(4-Fluorophenyl)-1-[3-[[2-(3-methoxyphenyl)ethyl](prop-2-en-1-yl)amino]propyl]-1,3-dihydroisobenzofuran-5-carbonitrile,
1-(4-Fluorophenyl)-1-[3-[[2-(2-methoxyphenyl)ethyl](prop-2-en-1-yl)amino]propyl]-1,3-dihydroisobenzofuran-5-carbonitrile,
1-[3-[[2-(2,5-Dimethoxyphenyl)ethyl]methylamino]propyl]-1-(4-fluorophenyl)-1,3-dihydroisobenzofuran-5-carbonitrile,
1-[3-[[2-(2,5-Dimethoxyphenyl)ethyl](prop-2-en-1-yl)amino]propyl]-1-(4-fluorophenyl)-1,3-dihydroisobenzofuran-5-carbonitrile,
1-(4-Fluorophenyl)-1-[3-[[2-phenoxyethyl]methylamino]propyl]-1,3-dihydroisobenzofuran-5-carbonitrile,
1-[3-[[2-(1*H*-Indolyl-3-yl)ethyl](prop-2-en-1-yl)amino]propyl]-1-(4-fluorophenyl)-1,3-dihydroisobenzofuran-5-carbonitrile,
1-(4-Fluorophenyl)-1-[3-[[2-phenoxyethyl](prop-2-en-1-yl)amino]propyl]-1,3-dihydroisobenzofuran-5-carbonitrile,
1-(4-Fluorophenyl)-1-[3-[[3-(2-methoxyphenyl)propyl]methylamino]propyl]-1,3-dihydroisobenzofuran-5-carbonitrile,
1-(4-Fluorophenyl)-1-[3-[[3-(2-methoxyphenyl)propyl](prop-2-en-1-yl)amino]propyl]-1,3-dihydroisobenzofuran-5-carbonitrile,
1-(4-Fluorophenyl)-1-[3-[[3-(3-methoxyphenyl)propyl](prop-2-en-1-yl)amino]propyl]-1,3-dihydroisobenzofuran-5-carbonitrile,
1-(4-Fluorophenyl)-1-[3-[[3-(2-methoxyphenoxy)propyl]methylamino]propyl]-1,3-dihydroisobenzofuran-5-carbonitrile,
1-(4-Fluorophenyl)-1-[3-[[3-(2-methoxyphenoxy)propyl](prop-2-en-1-yl)amino]propyl]-1,3-dihydroisobenzofuran-5-carbonitrile,
1-(4-Fluorophenyl)-1-[3-[[3-(3-methoxyphenoxy)propyl]methylamino]propyl]-1,3-dihydroisobenzofuran-5-carbonitrile,
1-(4-Fluorophenyl)-1-[3-[[3-(3-methoxyphenoxy)propyl](prop-2-en-1-yl)amino]propyl]-1,3-dihydroisobenzofuran-5-carbonitrile,
1-[3-[(2-Benzyloxyethyl)methylamino]propyl]-1-(4-fluorophenyl)-1,3-dihydroisobenzofuran-5-carbonitrile,
1-[3-[(2-Benzyloxyethyl)(prop-2-en-1-yl)amino]propyl]-1-(4-fluorophenyl)-1,3-dihydroisobenzofuran-5-carbonitrile,
1-[3-[[3-(1*H*-Indolyl-3-yl)propyl](prop-2-en-1-yl)amino]propyl]-1-(4-fluorophenyl)-1,3-dihydroisobenzofuran-5-carbonitrile,
1-[3-[[3-(1*H*-Indolyl-3-yl)propyl](2-propynyl)amino]propyl]-1-(4-fluorophenyl)-1,3-dihydroisobenzofuran-5-carbonitrile,
1-[3-[[3-(1*H*-Indolyl-3-yl)propyl]methylamino]propyl]-1-(4-fluorophenyl)-1,3-dihydroisobenzofuran-5-carbonitrile,
1-[3-[[2-(5-Methyl-1*H*-indol-3-yl)ethyl]methylamino]propyl]-1-(4-fluorophenyl)-1,3-dihydroisobenzofuran-5-carbonitrile,
1-[3-[[2-(7-Fluoro-1*H*-indol-3-yl)ethyl]methylamino]propyl]-1-(4-fluorophenyl)-1,3-dihydroisobenzofuran-5-carbonitrile,
5-Fluoro-1-[3-[[3-(5-methyl-1*H*-indol-3-yl)propyl]methylamino]propyl]-1-(4-fluorophenyl)-1,3-dihydroisobenzofuran,
5-Fluoro-1-[3-[[3-(7-fluoro-1*H*-indol-3-yl)propyl]methylamino]propyl]-1-(4-fluorophenyl)-1,3-dihydroisobenzofuran,
1-[3-[[3-(5-Methyl-1*H*-indol-3-yl)propyl]methylamino]propyl]-1-(4-fluorophenyl)-1,3-dihydroisobenzofuran-5-carbonitrile,
1-[3-[Ethyl[3-(1*H*-indol-3-yl)propyl]amino]propyl]-1-(4-fluorophenyl)-1,3-dihydroisobenzofuran-5-carbonitrile,
1-[3-[Ethyl[2-(5-methyl-1*H*-indol-3-yl)ethyl]amino]propyl]-1-(4-fluorophenyl)-1,3-dihydroisobenzofuran-5-carbonitrile,
1-[3-[[3-(7-Fluoro-1*H*-indol-3-yl)propyl]methylamino]propyl]-1-(4-fluorophenyl)-1,3-dihydroisobenzofuran-5-carbonitrile,
1-[3-[[3-(5-Fluoro-1*H*-indol-3-yl)propyl]methylamino]propyl]-1-(4-fluorophenyl)-1,3-dihydroisobenzofuran-5-carbonitrile,
1-[3-[Ethyl[2-(5-fluoro-1*H*-indol-3-yl)ethyl]amino]propyl]-1-(4-fluorophenyl)-1,3-dihydroisobenzofuran-5-carbonitrile,
1-[3-[Ethyl[2-(7-fluoro-1*H*-indol-3-yl)ethyl]amino]propyl]-1-(4-fluorophenyl)-1,3-dihydroisobenzofuran-5-carbonitrile,
1-[3-[[2-(5-Chloro-1*H*-indol-3-yl)ethyl]methylamino]propyl]-1-(4-fluorophenyl)-1,3-dihydroisobenzofuran-5-carbonitrile,
1-[3-[[3-(5-Chloro-1*H*-indol-3-yl)propyl]methylamino]propyl]-5-fluoro-1-(4-fluorophenyl)-1,3-dihydroisobenzofuran,
1-[3-[[4-(5-Methyl-1*H*-indol-3-yl)butyl]methylamino]propyl]-1-(4-fluorophenyl)-1,3-dihydroisobenzofuran-5-carbonitrile,
1-[3-[Ethyl[3-(5-methyl-1*H*-indol-3-yl)propyl]amino]propyl]-1-(4-fluorophenyl)-1,3-dihydroisobenzofuran-5-carbonitrile,
1-[3-[Ethyl[3-(7-fluoro-1*H*-indol-3-yl)propyl]amino]propyl]-1-(4-fluorophenyl)-1,3-dihydroisobenzofuran-5-carbonitrile,
1-[3-[Ethyl[3-(5-fluoro-1*H*-indol-3-yl)propyl]amino]propyl]-1-(4-fluorophenyl)-1,3-dihydroisobenzofuran-5-carbonitrile,
1-[3-[[3-(5-Chloro-1*H*-indol-3-yl)propyl]methylamino]propyl]-1-(4-fluorophenyl)-1,3-dihydroisobenzofuran-5-carbonitrile,
1-[3-[[2-(7-Chloro-1*H*-indol-3-yl)ethyl]ethylamino]propyl]-1-(4-fluorophenyl)-1,3-dihydroisobenzofuran-5-carbonitrile,
1-[3-[[2-(5-Chloro-1*H*-indol-3-yl)ethyl]ethylamino]propyl]-1-(4-fluorophenyl)-1,3-dihydroisobenzofuran-5-carbonitrile,
1-[3-[[2-(5,7-Difluoro-1*H*-indol-3-yl)ethyl]ethylamino]propyl]-1-(4-fluorophenyl)-1,3-dihydroisobenzofuran-5-carbonitrile,
1-[3-[[4-(5-Fluoro-1*H*-indol-3-yl)butyl]ethylamino]propyl]-1-(4-fluorophenyl)-1,3-dihydroisobenzofuran-5-carbonitrile,
1-[3-[[4-(5-Chloro-1*H*-indol-3-yl)butyl]methylamino]propyl]-1-(4-fluorophenyl)-1,3-dihydroisobenzofuran-5-carbonitrile,
1-[3-[[3-(5-Chloro-1*H*-indol-3-yl)propyl]ethylamino]propyl]-1-(4-fluorophenyl)-1,3-dihydroisobenzofuran-5-carbonitrile,
1-[3-[[3-(5,7-Difluoro-1*H*-indol-3-yl)propyl]ethylamino]propyl]-1-(4-fluorophenyl)-1,3-dihydroisobenzofuran-5-carbonitrile,
1-[3-[[2-(5-Bromo-1*H*-indol-3-yl)ethyl]methylamino]propyl]-1-(4-fluorophenyl)-1,3-dihydroisobenzofuran-5-carbonitrile,
1-[3-[[3-(5-Bromo-1*H*-indol-3-yl)propyl]methylamino]propyl]-1-(4-fluorophenyl)-1,3-dihydroisobenzofuran-5-carbonitrile,
1-[3-[[2-(5-Bromo-1*H*-indol-3-yl)ethyl]ethylamino]propyl]-1-(4-fluorophenyl)-1,3-dihydroisobenzofuran-5-carbonitrile,
1-[3-[[4-(5-Bromo-1*H*-indol-3-yl)butyl]methylamino]propyl]-1-(4-fluorophenyl)-1,3-dihydroisobenzofuran-5-carbonitrile,
1-[3-[[3-(5-Bromo-1*H*-indol-3-yl)propyl]methylamino]propyl]-1-(4-fluorophenyl)-1,3-dihydroisobenzofuran-5-carbonitrile,
1-[3-[Ethyl[2-(5-iodo-1*H*-indol-3-yl)ethyl]amino]propyl]-1-(4-fluorophenyl)-1,3-dihydroisobenzofuran-5-carbonitrile,
1-[3-[Ethyl[3-(5-iodo-1*H*-indol-3-yl)propyl]amino]propyl]-1-(4-fluorophenyl)-1,3-dihydroisobenzofuran-5-carbonitrile,
1-[2-[[4-(5-Chloro-1*H*-indol-3-yl)butyl]methylamino]ethyl]-1-(4-fluorophenyl)-1,3-dihydroisobenzofuran-5-carbonitrile,
1-[2-[[4-(5-Bromo-1*H*-indol-3-yl)butyl]methylamino]ethyl]-1-(4-fluorophenyl)-1,3-dihydroisobenzofuran-5-carbonitrile,
1-[4-[[2-(5,7-Difluoro-1*H*-indol-3-yl)ethyl]methylamino]butyl]-1-(4-fluorophenyl)-1,3-dihydroisobenzofuran-5-carbonitrile,
1-[4-[[2-(7-Chloro-1*H*-indol-3-yl)ethyl]methylamino]butyl]-1-(4-fluorophenyl)-1,3-dihydroisobenzofuran-5-carbonitrile,
1-[4-[[2-(5-Chloro-1*H*-indol-3-yl)ethyl]methylamino]butyl]-1-(4-fluorophenyl)-1,3-dihydroisobenzofuran-5-carbonitrile,
1-[4-[[2-(5-Bromo-1*H*-indol-3-yl)ethyl]methylamino]butyl]-1-(4-fluorophenyl)-1,3-dihydroisobenzofuran-5-carbonitrile,
1-[4-[[2-(5-Methyl-1*H*-indol-3-yl)ethyl]methylamino]butyl]-1-(4-fluorophenyl)-1,3-dihydroisobenzofuran-5-carbonitrile,
1-[4-[[2-(5-Iodo-1*H*-indol-3-yl)ethyl]methylamino]butyl]-1-(4-fluorophenyl)-1,3-dihydroisobenzofuran-5-carbonitrile,
1-[4-[[2-(5-*t*-Butyl-1*H*-indol-3-yl)ethyl]methylamino]butyl]-1-(4-fluorophenyl)-1,3-dihydroisobenzofuran-5-carbonitrile,
1-[4-[[2-(5-*i*-Propyl-1*H*-indol-3-yl)ethyl]methylamino]butyl]-1-(4-fluorophenyl)-1,3-dihydroisobenzofuran-5-carbonitrile,
1-[3-[[2-(5-Methyl-1*H*-indol-3-yl)ethyl](prop-2-en-1-yl)amino]propyl]-1-(4-fluorophenyl)-1,3-dihydroisobenzofuran-5-carbonitrile,
1-[3-[[2-(5-Fluoro-1*H*-indol-3-yl)ethyl](prop-2-en-1-yl)amino]propyl]-1-(4-fluorophenyl)-1,3-dihydroisobenzofuran-5-carbonitrile,
1-[3-[[2-(7-Fluoro-1*H*-indol-3-yl)ethyl](prop-2-en-1-yl)amino]propyl]-1-(4-fluorophenyl)-1,3-dihydroisobenzofuran-5-carbonitrile,
1-[3-[[3-(5-Fluoro-1*H*-indol-3-yl)propyl](prop-2-en-1-yl)amino]propyl]-1-(4-fluorophenyl)-1,3-dihydroisobenzofuran-5-carbonitrile,
1-[3-[[3-(7-Fluoro-1*H*-indol-3-yl)propyl](prop-2-en-1-yl)amino]propyl]-1-(4-fluorophenyl)-1,3-dihydroisobenzofuran-5-carbonitrile,
1-[3-[[2-(5-Chloro-1*H*-indol-3-yl)ethyl](prop-2-en-1-yl)amino]propyl]-1-(4-fluorophenyl)-1,3-dihydroisobenzofuran-5-carbonitrile,.
1-[3-[[2-(5,7-Difluoro-1*H*-indol-3-yl)ethyl]-propylamino]propyl]-1-(4-fluorophenyl)-1,3-dihydroisobenzofuran-5-carbonitrile,
1-[3-[[2-[5-(2-Propyl)-1*H*-indol-3-yl]ethyl]-2-propylamino]propyl]-1-(4-fluorophenyl)-1,3-dihydroisobenzofuran-5-carbonitrile,
1-[3-[[3-(4-Fluoro-7-methyl-1*H*-indol-3-yl)propyl](prop-2-en-1-yl)amino]propyl]-1-(4-fluorophenyl)-1,3-dihydroisobenzofuran-5-carbonitrile,
1-[3-[[2-(4-Chloro-7-methyl-1*H*-indol-3-yl)ethyl](prop-2-en-1-yl)amino]propyl]-1-(4-fluorophenyl)-1,3-dihydroisobenzofuran-5-carbonitrile,
1-[3-[[3-(5-Chloro-1*H*-indol-3-yl)propyl](prop-2-en-1-yl)amino]propyl]-1-(4-fluorophenyl)-1,3-dihydroisobenzofuran-5-carbonitrile,
1-[3-[[2-(5-Pyrrolo[3,2-*h*]-1*H*-quinolin-3-yl)ethyl](prop-2-en-1-yl)amino]propyl]-1-(4-fluorophenyl)-1,3-dihydroisobenzofuran-5-carbonitrile,
1-[3-[[3-(7-Fluoro-1*H*-indol-3-yl)propyl](2-furylmethyl)amino]propyl]-1-(4-fluorophenyl)-1,3-dihydroisobenzofuran-5-carbonitrile,
1-[3-[[4-(7-Carboxy-1*H*-indol-3-yl)butyl](prop-2-en-1-yl)amino]propyl]-1-(4-fluorophenyl)-1,3-dihydroisobenzofuran-5-carbonitrile,
1-[3-[[2-[5-Bromo-1*H*-indol-3-yl]ethyl]-propylamino]propyl]-1-(4-fluorophenyl)-1,3-dihydroisobenzofuran-5-carbonitrile,
1-[3-[[3-(1*H*-Indol-3-yl)propyl](2-phenoxyethyl)amino]propyl]-1-(4-fluorophenyl)-1,3-dihydroisobenzofuran-5-carbonitrile,
1-[3-[[2-(5-Methyl-1*H*-indol-3-yl)ethyl](2-phenoxyethyl)amino]propyl]-1-(4-fluorophenyl)-1,3-dihydroisobenzofuran-5-carbonitrile,
1-[3-[[2-(5-Fluoro-1*H*-indol-3-yl)ethyl](2-phenoxyethyl)amino]propyl]-1-(4-fluorophenyl)-1,3-dihydroisobenzofuran-5-carbonitrile,
1-[3-[[3-(5-Pyrrolo[3,2-*h*]-1*H*-quinolin-3-yl)propyl]-2-furylmethylamino]propyl]-1-(4-fluorophenyl)-1,3-dihydroisobenzofuran-5-carbonitrile,
1-[3-[[3-(5-Methyl-1*H*-indol-3-yl)propyl](2-phenoxyethyl)amino]propyl]-1-(4-fluorophenyl)-1,3-dihydroisobenzofuran-5-carbonitrile,
1-[3-[[3-(5-Fluoro-1*H*-indol-3-yl)propyl](2-phenoxyethyl)amino]propyl]-1-(4-fluorophenyl)-1,3-dihydroisobenzofuran-5-carbonitrile,
1-[3-[[2-(5,7-Difluoro-1*H*-indol-3-yl)ethyl](2-phenoxyethyl)amino]propyl]-1-(4-fluorophenyl)-1,3-dihydroisobenzofuran-5-carbonitrile,
1-[3-[[4-(5-Pyrrolo[3,2-*h*]-1*H*-quinolin-3-yl)butyl]-2-furylmethylamino]propyl]-1-(4-fluorophenyl)-1,3-dihydroisobenzofuran-5-carbonitrile,
1-[3-[2-Phenoxyethyl[2-[5-(2-propyl)-1*H*-indol-3-yl]ethyl]amino]propyl]-1-(4-fluorophenyl)-1,3-dihydroisobenzofuran-5-carbonitrile or
1-[3-[[2-(5-Bromo-1*H*-indol-3-yl)ethyl](2-phenoxyethyl)amino]propyl]-1-(4-fluorophenyl)-1,3-dihydroisobenzofuran-5-carbonitrile
or an acid addition salt thereof.

14. A pharmaceutical composition comprising a compound according to claims 1 to 13 or a pharmaceutically acceptable acid addition salt thereof and at least one pharmaceutically acceptable carrier or diluent.

15. The use of a compound according to claims 1 to 14 or a pharmaceutically acceptable acid addition salt thereof for the preparation of a medicament for the treatment of a disorder or disease responsive to the effect of 5-HT_{1A} receptors.

16. The use of a compound according to claim 15 wherein the medicament is for the treatment of depression, psychosis, anxiety disorders, panic disorder, obsessive compulsive disorder, impulse control disorder, alcohol abuse, aggression, ischaemia, senile dementia, cardiovascular disorders and social phobia.

## Patentansprüche

1. Isobenzofuran mit der allgemeinen Formel (I): 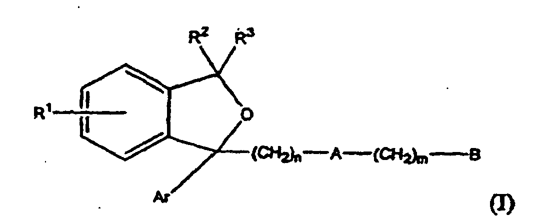 worin
R¹ Wasserstoff, Halogen, Trifluormethyl, Trifluormethylsulfonyloxy, C₁₋₆-Alkyl, C₂₋₆-Alkenyl, C₂₋₆-Alkinyl, C₃₋₈-Cycloalkyl, C₁₋₆-Alkoxy, Hydroxy, Formyl, Acyl, Amino, C₁₋₆-Alkylamino, C₂₋₁₂-Dialkylamino, Acylamino, C₁₋₆-Alkoxycarbonylamino, Aminocarbonylamino, C₁₋₆-Alkylaminocarbonylamino, C₂₋₁₂-Dialkylaminocarbonylamino, Nitro, Cyano, COOH oder COO-C₁₋₆-Alkyl ist;
R² und R³ jeweils unabhängig aus Wasserstoff, Trifluormethyl, C₁₋₆-Alkyl, C₂₋₆-Alkenyl, C₂₋₆-Alkinyl, C₃₋₈-Cycloalkyl und C₁₋₆-Alkoxy ausgewählt sind;
n 1, 2, 3, 4 oder 5 ist;
m 0 oder 1 ist;
A aus den folgenden Gruppen ausgewählt ist: 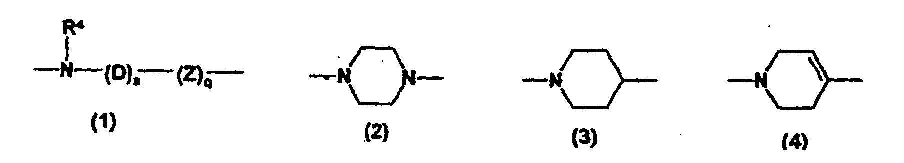 worin
Z O oder S ist;
s 0 oder 1 ist;
q 0 oder 1 ist;
R⁴ Wasserstoff, C₁₋₆-Alkyl, C₂₋₆-Alkenyl, C₂₋₆-Alkinyl, C₁₋₆-Alkyl-aryl oder C₁₋₆-Alkyl-O-aryl ist;
D eine aus verzweigtem oder geradkettigem C₁₋₆-Alkylen, C₂₋₆-Alkenylen oder C₂₋₆-Alkinylen ausgewählte Spacer-Gruppe ist;
B eine aus einer Gruppe der Formel (II), (III) und (IV) ausgewählte Gruppe ist 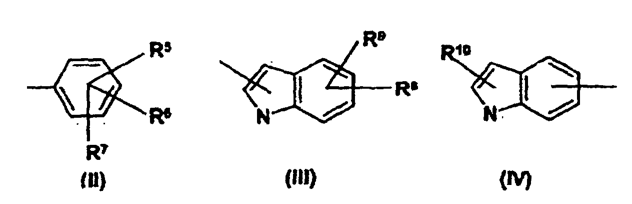 worin R⁵, R⁶, R⁷, R⁸, R⁹ und R¹⁰ jeweils unabhängig aus den R¹-Substituenten ausgewählt sind;
oder R⁸ und R⁹ zusammen einen kondensierten 5- oder 6-gliedrigen Ring bilden, der gegebenenfalls weitere Heteroatome enthält; und der resultierende Heterocyclus gegebenenfalls mit Substituenten substituiert ist, die aus den R¹-Substituenten ausgewählt sind;
oder zwei der Gruppen R⁵, R⁶ und R⁷ miteinander verbunden sind, um dadurch eine -O-(CH₂)ₚ-O- -Brücke zu bilden, worin p 1 oder 2 ist;
Ar und Aryl unabhängig aus der Gruppe ausgewählt sind, die aus Phenyl, 2-Thienyl, 3-Thienyl, 2-Furanyl, 3-Furanyl, 2-Pyrimidyl, 1-Indolyl, 2-Indolyl, 3-Indolyl, 1-Indol-2-onyl, 3-Indol-2-onyl, 2- oder 3-Benzofuranyl, 2- oder 3-Benzothiophenyl, 1-Naphthyl oder 2-Naphthyl besteht, jeweils gegebenenfalls substituiert mit Halogen, C₁₋₆-Alkyl, C₁₋₆-Alkoxy, C₁₋₆-Alkylthio, Hydroxy, C₁₋₆-Alkylsulfonyl, Cyano, Trifluormethyl, Trifluormethylsulfonyloxy, C₃₋₈-Cycloalkyl, C₃₋₈-Cycloalkyl-C₁₋₆-alkyl, Nitro, Amino, C₁₋₆-Alkylamino, C₂₋₁₂-Dialkylamino, Acylamino oder Alkylenodioxy;
seine Enantiomere und ein pharmazeutisch akzeptables Säureadditionssalz davon.

2. Verbindung gemäß Anspruch 1, **dadurch gekennzeichnet, daß** R⁴ Methyl, Ethyl, Propyl, 2-Propen-1-yl, 2-Furylmethyl oder 2-Phenoxyethyl ist.

3. Verbindung gemäß Anspruch 1, **dadurch gekennzeichnet**, das wenigstens eines aus R⁵, R⁶ und R⁷ Methoxy ist.

4. Verbindung gemäß Anspruch 1, **dadurch gekennzeichnet, daß** Formel (II) eine Benzodioxan-Gruppe oder eine 1,2-Methylendioxybenzol-Gruppe ist.

5. Verbindung gemäß Anspruch 1, **dadurch gekennzeichnet, daß** Formel (III) ein 3-Indolyl ist.

6. Verbindung gemäß Anspruch 5, **dadurch gekennzeichnet, daß** das 3-Indolyl in der 5-Position mit Methyl, Fluor, Chlor, Brom, Iod, t-Butyl oder i-Propyl substituiert ist oder in der 7-Position mit Fluor, Chlor oder Carboxy substituiert ist; oder mit 5,7-Difluor, 4-Fluor-7-methyl oder 4-Chlor-7-methyl disubstituiert ist oder die zwei Substituenten zusammen einen an das 3-Indolyl kondensierten Pyridyl-Ring bilden.

7. Verbindung gemäß Anspruch 1, **dadurch gekennzeichnet, daß** Formel (IV) eine 4-Indolyl- oder 5-Indolyl-Gruppe ist.

8. Verbindung gemäß einem der Ansprüche 1 bis 7, **dadurch gekennzeichnet, daß** Ar Phenyl oder mit Halogen oder CF₃ substituiertes Phenyl ist.

9. Verbindung gemäß einem der Ansprüche 1 bis 8, **dadurch gekennzeichnet, daß** R¹ H, CN oder F in der 5-Position der Isobenzofuran-Gruppe ist.

10. Verbindung gemäß einem der Ansprüche 1 bis 9, **dadurch gekennzeichnet, daß** R² und R³ aus Wasserstoff oder Methyl ausgewählt sind.

11. Verbindung gemäß einem der Ansprüche 1 bis 10, **dadurch gekennzeichnet, daß** n = 2, 3 oder 4 ist.

12. Verbindung gemäß einem der Ansprüche 1 bis 11, **dadurch gekennzeichnet, daß** m = 0 ist.

13. Verbindung gemäß Anspruch 1, die:
(-)-1-[3-[[4-(1,4-Benzodioxan-5-yl)butyl]methylamino]-propyl]-1-(4-fluorphenyl)-1,3-dihydroisobenzofuran-5-carbonitril,
1-[3-[[3-(1,4-Benzodioxan-5-yl)propyl]methylamino]-propyl]-1-(4-fluorphenyl)-1,3-dihydroisobenzofuran-5-carbonitriloxalat,
1-[3-[[2-(1,4-Benzodioxan-5-yl)ethyl]methylamino]-propyl]-1-(4-fluorphenyl)-1,3-dihydroisobenzofuran-5-carbonitriloxalat,
1- [3-[[1,4-Benzodioxan-5-ylmethyl]methylamino]propyl]-1-(4-fluorphenyl)-1,3-dihydroisobenzofuran-5-carbonitriloxalat,
1- (4-Fluorphenyl)-1-[3-[4-(2-methoxyphenyl)piperazinyl]-propyl]-1,3-dihydroisobenzofuran-5-carbonitril,
1-(4-Fluorphenyl)-1-[3-[methyl[2-(2-methoxyphenoxy)-ethyl]amino]propyl]-1,3-dihydroisobenzofuran-5-carbonitril,
1-(4-Fluorphenyl)-1-[3-[methyl[2-(3-methoxyphenoxy)-ethyl]amino]propyl]-1,3-dihydroisobenzofuran-5-carbonitril,
(S)-1-[3-[[4-(1H-Indol-3-yl)butyl]methylamino]propyl]-1-(4-fluorphenyl)-1,3-dihydroisobenzofuran-5-carbonitril,
1- [3-[[4-(1H-Indol-3-yl]butyl]methylamino]propyl]-1-phenyl-1,3-dihydroisobenzofuran,
(S)-1-[3-[[3-(1H-Indol-3-yl)propyl]methylamino]propyl]-1-(4-fluorphenyl)-1,3-dihydroisobenzofuran-5-carbonitril,
1-[3-[[3-(1H-Indol-3-yl)propyl]methylamino]propyl]-1-phenyl-1,3-dihydroisobenzofuran,
5-[3-[[3-(1-Phenyl-1,3-dihydroisobenzofuran-1-yl)-propyl]methylamino]propyl]-1,4-benzodioxan,
5-[3-[[3-(3-Chlorphenyl)-1,3-dihydroisobenzofuran-1-yl]-propyl]methylamino]propyl]-1,4-benzodioxan,
5-[3-[[3-[1-(4-Fluorphenyl)-1,3-dihydroisobenzofuran-1-yl]propyl]methylamino]propyl]-1,4-benzodioxan,
5-[3-[[3-[1-(3-Trifluormethylphenyl)-1,3-dihydroisobenzofuran-1-yl]propyl]methylamino]propyl]-1,4-benzodioxan,
1-[3-[[3-(1,4-Benzodioxan-5-yl)propyl]methylamino]-propyl]-1-(4-chlorphenyl)-1,3-dihydroisobenzofuran-5-carbonitril,
1-[3-[4-(1H-Indol-4-yl]piperazinyl]propyl]-1-(4-fluorphenyl)-1,3-dihydroisobenzofuran-5-carbonitril,
1-[3-[4-(1H-Indol-5-yl)piperazinyl]propyl]-1-(4-fluorphenyl)-1,3-dihydroisobenzofuran-5-carbonitril,
1-[3-[4-(1H-Indol-3-yl)piperidinyl]propyl]-1-(4-fluorphenyl)-1,3-dihydroisobenzofuran-5-carbonitril,
5-[3-[[3-[5-Fluor-1-(4-fluorphenyl)-1,3-dihydroisobenzofuran-1-yl]propyl]methylamino]propyl]-1,4-benzodioxan,
1-3-[[2-(1H-Indolyl-3-yl)ethyl]methylamino]propyl]-1-(4-fluorphenyl)-1,3-dihydroisobenzofuran-5-carbonitril,
1-(4-Fluorphenyl)-1-[3-[[2-(3-methoxyphenyl)ethyl]-methylamino]propyl]-1,3-dihydroisobenzofuran-5-carbonitril,
1-(4-Fluorphenyl)-1-[3-[[2-(3-methoxyphenyl)ethyl](prop-2-en-1-yl)amino]propyl]-1,3-dihydroisobenzofuran-5-carbonitril,
1-(4-Fluorphenyl)-1-[3-[[2-(2-methoxyphenyl)ethyl](prop-2-en-1-yl)amino]propyl]-1,3-dihydroisobenzofuran-5-carbonitril,
1-[3-[[2-(2,5-Dimethoxyphenyl)ethyl]methylamino]propyl]-1-(4-fluorphenyl)-1,3-dihydroisobenzofuran-5-carbonitril,
1-[3-[[2-(2,5-Dimethoxyphenyl)ethyl](prop-2-en-1-yl)amino]propyl]-1-(4-fluorphenyl)-1,3-dihydroisobenzofuran-5-carbonitril,
1-(4-Fluorphenyl)-1-[3-[[2-phenoxyethyl]methylamino]-propyl]-1,3-dihydroisobenzofuran-5-carbonitril,
1-[3-[[2-(1H-Indolyl-3-yl)ethyl](prop-2-en-1-yl)amino]-propyl]-1-(4-fluorphenyl)-1,3-dihydroisobenzofuran-5-carbonitril,
1-(4-Fluorphenyl)-1-[3-[[2-phenoxyethyl](prop-2-en-1-yl)amino]propyl]-1,3-dihydroisobenzofuran-5-carbonitril,
1-(4-Fluorphenyl)-1-[3-[[3-(2-methoxyphenyl)-propyl]methylamino]propyl]-1,3-dihydroisobenzofuran-5-carbonitril,
1-(4-Fluorphenyl)-1-[3-[[3-(2-methoxyphenyl)-propyl](prop-2-en-1-yl)amino]propyl]-1,3-dihydroisobenzofuran-5-carbonitril,
1-(4-Fluorphenyl)-1-[3-[[3-(3-methoxyphenyl)propyl]-(prop-2-en-1-yl)amino]propyl]-1,3-dihydroisobenzofuran-5-carbonitril,
1-(4-Fluorphenyl)-1-[3-[[3-(2-methoxyphenoxy)propyl]-methylamino]propyl]-1,3-dihydroisobenzofuran-5-carbonitril,
1-(4-Fluorphenyl)-1-[3-[[3-(2-methoxyphenoxy)propyl]-(prop-2-en-1-yl)amino]propyl]-1,3-dihydroisobenzofuran-5-carbonitril,
1-(4-Fluorphenyl)-1-[3-[[3-(3-methoxyphenoxy)propyl]-methylamino]propyl]-1,3-dihydroisobenzofuran-5-carbonitril,
1-(4-Fluorphenyl)-1-[3-[[3-(3-methoxyphenoxy)propyl]-(prop-2-en-1-yl)amino]propyl]-1,3-dihydroisobenzofuran-5-carbonitril,
1-[3-[(2-Benzyloxyethyl]methylamino]propyl]-1-(4-fluorphenyl)-1,3-dihydroisobenzofuran-5-carbonitril,
1-[3-[(2-Benzyloxyethyl)(prop-2-en-1-yl)amino]propyl]-1-(4-fluorphenyl)-1,3-dihydroisobenzofuran-5-carbonitril,
1-[3-[[3-(1H-Indolyl-3-yl)propyl](prop-2-en-1-yl)amino]propyl]-1-(4-fluorphenyl)-1,3-dihydroisobenzofuran-5-carbonitril,
1-[3-[[3-(1H-Indolyl-3-yl)propyl](2-propinyl)amino]propyl]-1-(4-fluorphenyl)-1,3-dihydroisobenzofuran-5-carbonitril,
1-[3-[[3-(1H-Indolyl-3-yl)propyl]methylamino]propyl]-1-(4-fluorphenyl)-1,3-dihydroisobenzofuran-5-carbonitril,
1-[3-[[2-(5-Methyl-1H-indol-3-yl)ethyl]-methylamino]propyl]-1-(4-fluorphenyl)-1,3-dihydroisobenzofuran-5-carbonitril,
1-[3-[[2-(7-Fluor-1H-indol-3-yl)ethyl]-methylamino]propyl]-1-(4-fluorphenyl)-1,3-dihydroisobenzofuran-5-carbonitril,
5-Fluor-1-[3-[[3-(5-methyl-1H-indol-3-yl)propyl]methylamino]propyl]-1-(4-fluorphenyl)-1,3-dihydroisobenzofuran,
5-Fluor-1-[3-[[3-(7-fluor-1H-indol-3-yl)propyl]methylamino]propyl]-1-(4-fluorphenyl)-1,3-dihydroisobenzofuran,
1-[3-[[3-(5-Methyl-1H-indol-3-yl)propyl]-methylamino]propyl]-1-(4-fluorphenyl)-1,3-dihydroisobenzofuran-5-carbonitril,
1-[3-[Ethyl[3-(1H-indol-3-yl)propyl]amino]propyl]-1-(4-fluorphenyl)-1,3-dihydroisobenzofuran-5-carbonitril,
1-[3-[Ethyl[2-(5-methyl-1H-indol-3-yl)ethyl]amino]propyl]-1-(4-fluorphenyl)-1,3-dihydroisobenzofuran-5-carbonitril,
1-[3-[[3-(7-Fluor-1H-indol-3-yl)propyl]methylamino]-propyl]-1-(4-fluorphenyl)-1,3-dihydroisobenzofuran-5-carbonitril,
1-[3-[[3-(5-Fluor-1H-indol-3-yl)propyl]methylamino]-propyl]-1-(4-fluorphenyl)-1,3-dihydroisobenzofuran-5-carbonitril,
1-[3-[Ethyl[2-(5-fluor-1H-indol-3-yl)ethyl]amino]-propyl]-1-(4-fluorphenyl)-1,3-dihydroisobenzofuran-5-carbonitril,
1-[3-[Ethyl[2-(7-fluor-1H-indol-3-yl)ethyl]amino]-propyl]-1-(4-fluorphenyl)-1,3-dihydroisobenzofuran-5-carbonitril,
1-[3-[[2-(5-Chlor-1H-indol-3-yl)ethyl]methylamino]-propyl]-1-(4-fluorphenyl)-1,3-dihydroisobenzofuran-5-carbonitril,
1-[3-[[3-(5-Chlor-1H-indol-3-yl]propyl]methylamino]-propyl]-5-fluor-1-(4-fluorphenyl)-1,3-dihydroisobenzofuran,
1-[3-[[4-(5-Methyl-1H-indol-3-yl)butyl]methylamino]-propyl]-1-(4-fluorphenyl)-1,3-dihydroisobenzofuran-5-carbonitril,
1-[3-[Ethyl[3-(5-methyl-1H-indol-3-yl)propyl]amino]-propyl]-1-(4-fluorphenyl)-1,3-dihydroisobenzofuran-5-carbonitril,
1-[3-[Ethyl[3-(7-fluor-1H-indol-3-yl)propyl]amino]-propyl]-1-(4-fluorphenyl)-1,3-dihydroisobenzofuran-5-carbonitril,
1-[3-[Ethyl[3-(5-fluor-1H-indol-3-yl)propyl]amino]-propyl]-1-(4-fluorphenyl)-1,3-dihydroisobenzofuran-5-carbonitril,
1-[3-[[3-(5-Chlor-1H-indol-3-yl)propyl]methylamino]-propyl]-1-(4-fluorphenyl)-1,3-dihydroisobenzofuran-5-carbonitril,
1-[3-[[2-(7-Chlor-1H-indol-3-yl)ethyl]ethylamino]-propyl]-1-(4-fluorphenyl)-1,3-dihydroisobenzofuran-5-carbonitril,
1-[3-[[2-(5-Chlor-1H-indol-3-yl)ethyl]ethylamino]-propyl]-1-(4-fluorphenyl)-1,3-dihydroisobenzofuran-5-carbonitril,
1-[3-[[2-(5,7-Difluor-1H-indol-3-yl)ethyl]ethylamino]-propyl]-1-(4-fluorphenyl)-1,3-dihydroisobenzofuran-5-carbonitril,
1-[3-[[4-(5-Fluor-1H-indol-3-yl)butyl]ethylamino]-propyl]-1-(4-fluorphenyl)-1,3-dihydroisobenzofuran-5-carbonitril,
1-[3-[[4-(5-Chlor-1H-indol-3-yl)butyl]methylamino]-propyl]-1-(4-fluorphenyl)-1,3-dihydroisobenzofuran-5-carbonitril,
1-[3-[[3-(5-Chlor-1H-indol-3-yl)propyl]ethylamino]-propyl]-1-(4-fluorphenyl)-1,3-dihydroisobenzofuran-5-carbonitril,
1-[3-[[3-(5,7-Difluor-1H-indol-3-yl)propyl]ethylamino]-propyl]-1-(4-fluorphenyl)-1,3-dihydroisobenzofuran-5-carbonitril,
1-[3-[[2-(5-Brom-1H-indol-3-yl)ethyl]methylamino]-propyl]-1-(4-fluorphenyl)-1,3-dihydroisobenzofuran-5-carbonitril,
1-[3-[[3-(5-Brom-1H-indol-3-yl)propyl]methylamino]-propyl]-1-(4-fluorphenyl)-1,3-dihydroisobenzofuran-5-carbonitril,
1-[3-[[2-(5-Brom-1H-indol-3-yl)ethyl]ethylamino]propyl]-1-(4-fluorphenyl)-1,3-dihydroisobenzofuran-5-carbonitril,
1-[3-[[4-(5-Brom-1H-indol-3-yl)butyl]methylamino]-propyl]-1-(4-fluorphenyl)-1,3-dihydroisobenzofuran-5-carbonitril,
1-[3-[[3-(5-Brom-1H-indol-3-yl)propyl]methylamino]-propyl]-1-(4-fluorphenyl)-1,3-dihydroisobenzofuran-5-carbonitril,
1-[3-[Ethyl[2-(5-iod-1H-indol-3-yl)ethyl]amino]propyl]-1-(4-fluorphenyl)-1,3-dihydroisobenzofuran-5-carbonitril,
1-[3-[Ethyl[3-(5-iod-1H-indol-3-yl)propyl]amino]propyl]-1-(4-fluorphenyl)-1,3-dihydroisobenzofuran-5-carbonitril,
1-[2-[[4-(5-Chlor-1H-indol-3-yl)butyl]methylamino]-ethyl]-1-(4-fluorphenyl)-1,3-dihydroisobenzofuran-5-carbonitril,
1-[2-[[4-(5-Brom-1H-indol-3-yl)butyl]methylamino]ethyl]-1-(4-fluorphenyl)-1,3-dihydroisobenzofuran-5-carbonitril,
1-[4-[[2-(5,7-Difluor-1H-indol-3-yl)ethyl]methylamino]-butyl]-1-(4-fluorphenyl)-1,3-dihydroisobenzofuran-5-carbonitril,
1-[4-[[2-(7-Chlor-1H-indol-3-yl)ethyl]methylamino]-butyl]-1-(4-fluorphenyl)-1,3-dihydroisobenzofuran-5-carbonitril,
1-[4-[[2-(5-Chlor-1H-indol-3-yl)ethyl]methylamino]-butyl]-1-(4-fluorphenyl)-1,3-dihydroisobenzofuran-5-carbonitril,
1-[4-[[2-(5-Brom-1H-indol-3-yl)ethyl]methylamino]butyl]-1-(4-fluorphenyl)-1,3-dihydroisobenzofuran-5-carbonitril,
1-[4-[[2-(5-Methyl-1H-indol-3-yl)ethyl]methylamino]-butyl]-1-(4-fluorphenyl)-1,3-dihydroisobenzofuran-5-carbonitril,
1-[4-[[2-(5-Iod-1H-indol-3-yl)ethyl)methylamino]butyl]-1-(4-fluorphenyl)-1,3-dihydroisobenzofuran-5-carbonitril,
1-[4-[[2-(5-t-Butyl-1H-indol-3-yl)ethyl]methylamino]-butyl]-1-(4-fluorphenyl)-1,3-dihydroisobenzofuran-5-carbonitril,
1-[4-[[2-(5-i-Propyl-1H-indol-3-yl)ethyl]methylamino]-butyl]-1-(4-fluorphenyl)-1,3-dihydroisobenzofuran-5-carbonitril,
1-[3-[[2-(5-Methyl-1H-indol-3-yl)ethyl](prop-2-en-1-yl)amino]propyl]-1-(4-fluorphenyl)-1,3-dihydroisobenzofuran-5-carbonitril,
1-[3-[[2-(5-Fluor-1H-indol-3-yl)ethyl](prop-2-en-1-yl)amino]propyl]-1-(4-fluorphenyl)-1,3-dihydroisobenzofuran-5-carbonitril,
1-[3-[[2-(7-Fluor-1H-indol-3-yl)ethyl](prop-2-en-1-yl)amino]propyl]-1-(4-fluorphenyl)-1,3-dihydroisobenzofuran-5-carbonitril,
1-[3-[[3-(5-Fluor-1H-indol-3-yl)propyl](prop-2-en-1-yl)amino]propyl)-1-(4-fluorphenyl)-1,3-dihydroisobenzofuran-5-carbonitril,
1-[3-[[3-(7-Fluor-1H-indol-3-yl)propyl](prop-2-en-1-yl)amino]propyl]-1-(4-fluorphenyl)-1,3-dihydroisobenzofuran-5-carbonitril,
1-[3-[[2-(5-Chlor-1H-indol-3-yl)ethyl](prop-2-en-1-yl)amino]propyl]-1-(4-fluorphenyl)-1,3-dihydroisobenzofuran-5-carbonitril,
1-[3-[[2-(5,7-Difluor-1H-indol-3-yl)ethyl]propylamino]-propyl]-1-(4-fluorphenyl)-1,3-dihydroisobenzofuran-5-carbonitril,
1-[3-[[2-[5-(2-Propyl)-1H-indol-3-yl]ethyl]-2-propylamino]propyl]-1-(4-fluorphenyl)-1,3-dihydroisobenzofuran-5-carbonitril,
1-[3-[[3-(4-Fluor-7-methyl-1H-indol-3-yl)propyl](prop-2-en-1-yl)amino]propyl]-1-(4-fluorphenyl)-1,3-dihydroisobenzofuran-5-carbonitril,
1-[3-[[2-(4-Chlor-7-methyl-1H-indol-3-yl)ethyl](prop-2-en-1-yl)amino]propyl]-1-(4-fluorphenyl)-1,3-dihydroisobenzofuran-5-carbonitril,
1-[3-[[3-(5-Chlor-1H-indol-3-yl)propyl](prop-2-en-1-yl)amino]propyl]-1-(4-fluorphenyl)-1,3-dihydroisobenzofuran-5-carbonitril,
1-[3-[[2-(5-Pyrrolo[3,2-h]-1H-chinolin-3-yl)ethyl](prop-2-en-1-yl)amino]propyl]-1-(4-fluorphenyl)-1,3-dihydroisobenzofuran-5-carbonitril,
1-[3-[[3-(7-Fluor-1H-indol-3-yl)propyl](2-furylmethyl)amino]propyl]-1-(4-fluorphenyl)-1,3-dihydroisobenzofuran-5-carbonitril,
1-[3-[[4-(7-Carboxy-1H-indol-3-yl)butyl](prop-2-en-1-yl)amino]propyl)-1-(4-fluorphenyl)-1,3-dihydroisobenzofuran-5-carbonitril,
1-[3-[[2-(5-Brom-1H-indol-3-yl)ethyl]-propylamino]propyl]-1-(4-fluorphenyl)-1,3-dihydroisobenzofuran-5-carbonitril,
1-[3-[[3-(1H-Indol-3-yl)propyl](2-phenoxyethyl)amino]propyl]-1-(4-fluorphenyl)-1,3-dihydroisobenzofuran-5-carbonitril,
1-[3-[[2-(5-Methyl-1H-indol-3-yl)ethyl](2-phenoxyethyl)amino]propyl]-1-(4-fluorphenyl)-1,3-dihydroisobenzofuran-5-carbonitril,
1-[3-[[2-(5-Fluor-1H-indol-3-yl)ethyl](2-phenoxyethyl)amino]propyl]-1-(4-fluorphenyl)-1,3-dihydroisobenzofuran-5-carbonitril,
1-[3-[[3-(5-Pyrrolo[3,2-h]-1H-chinolin-3-yl)propyl]-2-furylmethylamino]propyl]-1-(4-fluorphenyl)-1,3-dihydroisobenzofuran-5-carbonitril,
1-[3-[[3-(5-Methyl-1H-indol-3-yl)propyl](2-phenoxyethyl)amino]propyl]-1-(4-fluorphenyl)-1,3-dihydroisobenzofuran-5-carbonitril,
1-[3-[[3-(5-Fluor-1H-indol-3-yl)propyl](2-phenoxyethyl)amino]propyl]-1-(4-fluorphenyl)-1,3-dihydroisobenzofuran-5-carbonitril,
1-[3-[[2-(5,7-Difluor-1H-indol-3-yl)propyl](2-phenoxyethyl)amino]propyl]-1-(4-fluorphenyl)-1,3-dihydroisobenzofuran-5-carbonitril,
1-[3-[(4-(5-Pyrrolo[3,2-h]-1H-chinolin-3-yl)butyl]-2-furylmethylamino]propyl]-1-(4-fluorphenyl)-1,3-dihydroisobenzofuran-5-carbonitril,
1-[3-[2-Phenoxyethyl[2-[5-(2-propyl)-1H-indol-3-yl]ethyl]amino]propyl]-1-(4-fluorphenyl)-1,3-dihydroisobenzofüran-5-carbonitril oder 1-[3-[[2-(5-Brom-1H-indol-3-yl)ethyl](2-phenoxyethyl)amino]propyl]-1-(4-fluorphenyl)-1,3-dihydroisobenzofuran-5-carbonitril,
oder ein Säureadditionssalz davon ist.

14. Pharmazeutische Zusammensetzung, die eine Verbindung gemäß Ansprüchen 1 bis 13 oder ein pharmazeutisch akzeptables Säureadditionssalz davon und wenigstens einen pharmazeutisch akzeptablen Träger oder Verdünnungsstoff umfaßt.

15. Verwendung einer Verbindung gemäß Ansprüchen 1 bis 14 oder eines pharmazeutisch akzeptablen Säureadditionssalzes davon zur Herstellung eines Medikaments zur Behandlung einer Störung oder Krankheit, die auf die Wirkung von 5-HT_{1A}-Rezeptoren anspricht.

16. Verwendung einer Verbindung gemäß Anspruch 15, worin das Medikament zur Behandlung von Depression, Psychose, Angststörungen, Panikstörung, Zwangsstörung, Störung der Impulskontrolle, Alkoholmißbrauch, Aggression, Ischämie, Altersdemenz, kardiovaskulären Störungen und sozialer Phobie ist.

## Revendications

1. Isobenzofurane ayant la formule générale I: 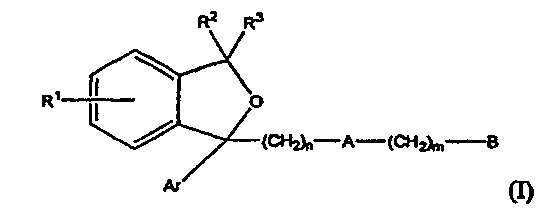 dans laquelle
R¹ est un atome d'hydrogène ou un reste halogéno, trifluorométhyle, trifluorométhylsulfonyloxy, alkyle en C₁-C₆, alcényle en C₂-C₆, alcynyle en C₂-C₆, cycloalkyle en C₃-C₈, alcoxy en C₁-C₆, hydroxy, formyle, acyle, amino, alkylamino en C₁-C₆, dialkylamino en C₂-C₁₂, acylamino, (alcoxy en C₁-C₆)carbonylamino, aminocarbonylamino, (alkyl en C₁-C₆)aminocarbonylamino, (dialkyl en C₂-C₁₂)aminocarbonylamino, nitro, cyano, COOH ou COO-(alkyle en C₁-C₆);
R² et R³ sont choisis chacun indépendamment parmi un atome d'hydrogène et un reste trifluorométhyle, alkyle en C₁-C₆, alcényle en C₂-C₆, alcynyle en C₂-C₆, cycloalkyle en C₃-C₈ et alcoxy en C₁-C₆;
n est 1, 2, 3, 4 ou 5;
m est 0 ou 1;
A est choisi parmi les groupes suivants: 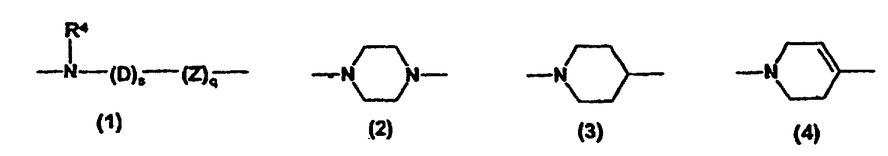
dans lesquels
Z est O ou S;
s est 0 ou 1;
q est 0 ou 1;
R⁴ est un atome d'hydrogène ou un reste alkyle en C₁-C₆, alcényle en C₂-C₆, alcynyle en C₂-C₆, (alkyle en C₁-C₆)-Aryl ou (alkyl en C₁-C₆)-O-Aryl;
D est un groupe espaceur choisi parmi des restes alkylène en C₁-C₆, alcénylène en C₂-C₆ et alcynylène en C₂-C₆, linéaires ou ramifiés;
B est un groupe choisi parmi les groupes de formule (II), (III) et (IV) 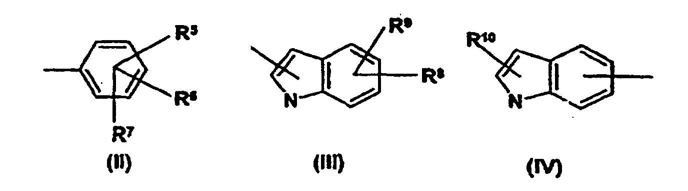 dans lesquels R⁵, R⁶, R⁷, R⁸, R⁹ et R¹⁰ sont choisis chacun indépendamment parmi les substituants R¹;
ou R⁸ et R⁹ forment ensemble un cycle condensé de 5 ou 6 chaînons contenant éventuellement d'autres hétéroatomes; et l'hétérocycle obtenu est éventuellement substitué par des substituants choisis parmi les substituants R¹;
ou deux des groupes R⁵, R⁶ et R⁷ sont liés entre eux en formant un pont -O-(CH₂)ₚ-O- où p est 1 ou 2;
Ar et Aryl sont choisis indépendamment dans le groupe constitué par les restes phényle, 2-thiényle, 3-thiényle, 2-furanyle, 3-furanyle, 2-pyrimidyle, 1-indolyle, 2-indolyle, 3-indolyle, 1-indol-2-onyle, 3-indol-2-onyle, 2- ou 3-benzofuranyle, 2- ou 3-benzothiophényle, 1-naphtyle ou 2-naphtyle, qui sont chacun éventuellement substitués par un reste halogéno, alkyle en C₁-C₆, alcoxy en C₁-C₆, alkylthio en C₁-C₆, hydroxy, alkylsulfonyle en C₁-C₆, cyano, trifluorométhyle, trifluorométhylsulfonyloxy, cycloalkyle en C₃-C₈, (cycloalkyl en C₃-C₈)-(alkyle en C₁-C₆), nitro, amino, alkylamino en C₁-C₆, dialkylamino en C₂-C₁₂, acylamino ou alkylènedioxy;
ses énantiomères, et ses sels d'addition d'acide pharmaceutiquement acceptables.

2. Composé selon la revendication 1, **caractérisé en ce que** R⁴ est un reste méthyle, éthyle, propyle, 2-propén-1-yle, 2-furylméthyle, 2-phénoxyéthyle.

3. Composé selon la revendication 1, **caractérisé en ce qu'**au moins l'un des R⁵, R⁶ et R⁷ est un reste méthoxy.

4. Composé selon la revendication 1, **caractérisé en ce que** la formule (II) représente un groupe benzodioxane ou un groupe 1,2-méthylènedioxybenzène.

5. Composé selon la revendication 1, **caractérisé en ce que** la formule (III) représente un groupe 3-indolyle.

6. Composé selon la revendication 5, **caractérisé en ce que** le groupe 3-indolyle est substitué en position 5 par un reste méthyle, fluoro, chloro, bromo, iodo, t-butyle ou isopropyle, ou en position 7 par un reste fluoro, chloro ou carboxy; ou est disubstitué par 5,7-difluoro, 4-fluoro-7-méthyle ou 4-chloro-7-méthyle, ou les deux substituants forment ensemble un cycle pyridyle condensé avec le 3-indolyle.

7. Composé selon la revendication 1, **caractérisé en ce que** la formule (IV) représente un groupe 4-indolyle ou 5-indolyle.

8. Composé selon l'une quelconque des revendications 1 - 7, **caractérisé en ce que** Ar est un reste phényle ou phényle substitué par halogéno ou CF₃.

9. Composé selon l'une quelconque des revendications 1 - 8, **caractérisé en ce que** R¹ est H, CN ou F en position 5 du groupe isobenzofurane.

10. Composé selon l'une quelconque des revendications 1 - 9, **caractérisé en ce que** R² et R³ sont choisis parmi un atome d'hydrogène et un groupe méthyle.

11. Composé selon l'une quelconque des revendications 1 - 10, **caractérisé en ce que** n = 2, 3 ou 4.

12. Composé selon l'une quelconque des revendications 1 - 11, **caractérisé en ce que** m = 0.

13. Composé selon la revendication 1, qui est
le (-)-1-[3-[[4-(1,4-benzodioxane-5-yl)butyl]méthylamino]propyl]-1-(4-fluorophényl)-1,3-dihydroisobenzofurane-5-carbonitrile,
l'oxalate de 1-[3-[[3-(1,4-benzodioxane-5-yl)propyl]méthylamino]propyl]-1-(4-fluorophényl)-1,3-dihydroisobenzofurane-5-carbonitrile,
l'oxalate de 1-[3-[[2-(1,4-benzodioxane-5-yl)éthyl]méthylamino]propyl]-1-(4-fluorophényl)-1,3-dihydroisobenzofurane-5-carbonitrile,
l'oxalate de 1-[3-[[1,4-benzodioxane-5-ylméthyl]méthylamino]propyl]-1-(4-fluorophényl)-1,3-dihydroisobenzofurane-5-carbonitrile,
le 1-(4-fluorophényl)-1-[3-[4-(2-méthoxyphényl)pipérazinyl]propyl]-1,3-dihydroisobenzofurane-5-carbonitrile,
le 1-(4-fluorophényl)-1-[3-[méthyl[2-(2-méthoxyphénoxy)éthyl]amino]propyl]-1,3-dihydroisobenzofurane-5-carbonitrile,
le 1-(4-fluorophényl)-1-[3-[méthyl[2-(3-méthoxyphénoxy)éthyl]amino]propyl]-1,3-dihydroisobenzofurane-5-carbonitrile,
le (S)-1-[3-[[4-(1H-indol-3-yl)butyl]méthylamino]propyl]-1-(4-fluorophényl)-1,3-dihydroisobenzofurane-5-carbonitrile,
le 1-[3-[[4-(1H-indol-3-yl)butyl]méthylamino]propyl]-1-phényl-1,3-dihydroisobenzofurane,
le (S)-1-[3-[[3-(1H-indol-3-yl)propyl]méthylamino]propyl]-1-(4-fluorophényl)-1,3-dihydroisobenzofurane-5-carbonitrile,
le 1-[3-[[3-(1H-indol-3-yl)propyl]méthylamino]propyl]-1-phényl-1,3-dihydroisobenzofurane,
le 5-[3-[[3-(1-phényl-1,3-dihydroisobenzofurane-1-yl)propyl]méthylamino]propyl]-1,4-benzodioxane,
le 5-[3-[[3-[1-(3-chlorophényl)-1,3-dihydroisobenzofurane-1-yl]propyl]méthylamino]propyl]-1,4-benzodioxane,
le 5-[3-[[3-[1-(4-fluorophényl)-1,3-dihydroisobenzofurane-1-yl]propyl]méthylamino]propyl]-1,4-benzodioxane;
le 5-[3-[[3-[1-(3-trifluorométhylphényl)-1,3-dihydroisobenzofurane-1-yl]propyl]-méthylamino]propyl]-1,4-benzodioxane,
le 1-[3-[[3-(1,4-benzodioxane-5-yl)propyl]méthylamino]propyl]-1-(4-chlorophényl)-1,3-dihydroisobenzofurane-5-carbonitrile,
le 1-[3-[4-(1H-indol-4-yl)pipérazinyl]propyl]-1-(4-fluorophényl)-1,3-dihydroisobenzofurane-5-carbonitrile,
le 1-[3-[4-(1H-indol-5-yl)pipérazinyl]propyl]-1-(4-fluorophényl)-1,3-dihydroisobenzofurane-5-carbonitrile,
le 1-[3-[4-(1H-indol-3-yl)pipéridinyl]propyl]-1-(4-fluorophényl)-1,3-dihydroisobenzofurane-5-carbonitrile,
le 5-[3-[[3-[-5-fluoro-1-(4-fluorophényl)-1,3-dihydroisobenzofurane-1-yl]propyl]-méthylamino]propyl]-1,4-benzodioxane,
le 1-[3-[[2-(1H-indolyl-3-yl)éthyl]méthylamino]propyl]-1-(4-fluorophényl)-1,3-dihydroisobenzofurane-5-carbonitrile,
le 1-(4-fluorophényl)-1-[3-[[2-(3-méthoxyphényl)éthyl]méthylamino]propyl]-1,3-dihydroisobenzofurane-5-carbonitrile,
le 1-(4-fluorophényl)-1-[3-[[2-(3-méthoxyphényl)éthyl](prop-2-én-1-yl)amino]-propyl]-1,3-dihydroisobenzofurane-5-carbonitrile,
le 1-(4-fluorophényl)-1-[3-[[2-(2-méthoxyphényl)éthyl](prop-2-én-1-yl)amino]-propyl]-1,3-dihydroisobenzofurane-5-carbonitrile,
le 1-[3-[[2-(2,5-diméthoxyphényl)éthyl]méthylamino]propyl]-1-(4-fluorophényl)-1,3-dihydroisobenzofurane-5-carbonitrile,
le 1-[3-[[2-(2,5-diméthoxyphényl)éthyl](prop-2-én-1-yl)amino]propyl]-1-(4-fluorophényl)-1,3-dihydroisobenzofurane-5-carbonitrile,
le 1-(4-fluorophényl)-1-[3-[[2-phénoxyéthyl]méthylamino]propyl]-1,3-dihydroisobenzofurane-5-carbonitrile,
le 1-[3-[[2-(1H-indolyl-3-yl)éthyl](prop-2-én-1-yl)amino]propyl]-1-(4-fluorophényl)-1,3-dihydroisobenzofurane-5-carbonitrile,
le 1-(4-fluorophényl)-1-[3-[[2-phénoxyéthyl](prop-2-én-1-yl)amino]propyl]-1,3-dihydroisobenzofurane-5-carbonitrile,
le 1-(4-fluorophényl)-1-[3-[[3-(2-méthoxyphényl)propyl]méthylamino]propyl]-1,3-dihydroisobenzofurane-5-carbonitrile,
le 1-(4-fluorophényl)-1-[3-[[3-(2-méthoxyphényl)propyl](prop-2-én-1-yl)amino]-propyl]-1,3-dihydroisobenzofurane-5-carbonitrile,
le 1-(4-fluorophényl)-1-[3-[[3-(3-méthoxyphényl)propyl](prop-2-én-1-yl)amino]-propyl]-1,3-dihydroisobenzofurane-5-carbonitrile,
le 1-(4-fluorophényl)-1-[3-[[3-(2-méthoxyphénoxy)propyl]méthylamino]propyl]-1,3-dihydroisobenzofurane-5-carbonitrile,
le 1-(4-fluorophényl)-1-[3-[[3-(2-méthoxyphénoxy)propyl](prop-2-én-1-yl)amino]-propyl]-1,3-dihydroisobenzofurane-5-carbonitrile,
le 1-(4-fluorophényl)-1-[3-[[3-(3-méthoxyphénoxy)propyl]méthylamino]propyl]-1,3-dihydroisobenzofurane-5-carbonitrile,
le 1-(4-fluorophényl)-1-[3-[[3-(3-méthoxyphénoxy)propyl]-prop-2-én-1-yl)amino]-propyl]-1,3-dihydroisobenzofurane-5-carbonitrile,
le 1-[3-[(2-benzyloxyéthyl)méthylamino]propyl]-1-(4-fluorophényl)-1,3-dihydroisobenzofurane-5-carbonitrile,
le 1-[3-[(2-benzyloxyéthyl)(prop-2-én-1-yl)amino]propyl]-1-(4-fluorophényl)-1,3-dihydroisobenzofurane-5-carbonitrile,
le 1-[3-[[3-( 1H-indolyl-3-yl)propyl](prop-2-én-1-yl)amino]propyl]-1-(4-fluorophényl)-1,3-dihydroisobenzofurane-5-carbonitrile,
le 1-[3-[[3-(1H-indolyl-3-yl)propyl](2-propynyl)amino]propyl]-1-(4-fluorophényl)-1,3-dihydroisobenzofurane-5-carbonitrile,
le 1-[3-[[3-(1H-indolyl-3-yl)propyl]méthylamino]propyl]-1-(4-fluorophényl)-1,3-dihydroisobenzofurane-5-carbonitrile,
le 1-[3-[[2-(5-méthyl-1H-indol-3-yl)éthyl]méthylamino]propyl]-1-(4-fluorophényl)-1,3-dihydroisobenzofurane-5-carbonitrile,
le 1-[3-[[2-(7-fluoro-1H-indol-3-yl)éthyl]méthylamino]propyl]-1-(4-fluorophényl)-1,3-dihydroisobenzofurane-5-carbonitrile,
le 5-fluoro-1-[3-[[3-(5-méthyl-1H-indol-3-yl)propyl]méthylamino]propyl]-1-(4-fluorophényl)-1,3-dihydroisobenzofurane,
le 5-fluoro-1-[3-[[3-(7-fluoro-1H-indol-3-yl)propyl]méthylamino]propyl]-1-(4-fluorophényl)-1,3-dihydroisobenzofurane,
le 1-[3-[[3-(5-méthyl-1H-indol-3-yl)propyl]méthylamino]propyl]-1-(4-fluorophényl)-1,3-dihydroisobenzofurane-5-carbonitrile,
le 1-[3-[éthyl[3-(1H-indol-3-yl)propyl]amino]propyl]-1-(4-fluorophényl)-1,3-dihydroisobenzofurane-5-carbonitrile,
le 1-[3-[éthyl[2-(5-méthyl-1H-indol-3-yl)éthyl]amino]propyl]-1-(4-fluorophényl)-1,3-dihydroisobenzofurane-5-carbonitrile,
le 1-[3-[[3-(7-fluoro-1H-indol-3-yl)propyl]méthylamino]propyl]-1-(4-fluorophényl)-1,3-dihydroisobenzofurane-5-carbonitrile,
le 1-[3-[[3-(5-fluoro-1H-indol-3-yl)propyl]méthylamino]propyl]-1-(4-fluorophényl)-1,3-dihydroisobenzofurane-5-carbonitrile,
le 1-[3-[éthyl [2-(5-fluoro-1H-indol-3-yl)éthyl]amino]propyl]-1-(4-fluorophényl)-1,3-dihydroisobenzofurane-5-carbonitrile,
le 1-[3-[éthyl[2-(7-fluoro-1H-indol-3-yl)éthyl]amino]propyl]-1-(4-fluorophényl)-1,3-dihydroisobenzofurane-5-carbonitrile,
le 1-[3-[[2-(5-chloro-1H-indol-3-yl)éthyl]méthylamino]propyl]-1-(4-fluorophényl)-1,3-dihydroisobenzofurane-5-carbonitrile,
le 1-[3-[[3-(5-chloro-1H-indol-3-yl)propyl]méthylamino]propyl]-5-fluoro-1-(4-fluorophényl)-1,3-dihydroisobenzofurane,
le 1-[3-[[4-(5-méthyl-1H-indol-3-yl)butyl]méthylamino]propyl]-1-(4-fluorophényl)-1,3-dihydroisobenzofurane-5-carbonitrile,
le 1-[3-[éthyl[3-(5-méthyl-1H-indol-3-yl)propyl]amino]propyl]-1-(4-fluorophényl)-1,3-dihydroisobenzofurane-5-carbonitrile,
le 1-[3-[éthyl[3-(7-fluoro-1H indol-3-yl)propyl]amino]propyl]-1-(4-fluorophényl)-1,3-dihydroisobenzofurane-5-carbonitrile,
le 1-[3-[éthyl[3-(5-fluoro-1H-indol-3-yl)propyl]amino]propyl]-1-(4-fluorophényl)-1,3-dihydroisobenzofurane-5-carbonitrile,
le 1-[3-[[3-(5-chloro-1H-indol-3-yl)propyl]méthylamino]propyl]-1-(4-fluorophényl)-1,3-dihydroisobenzofurane-5-carbonitrile,
le 1-[3-[[2-(7-chloro-1H-indol-3-yl)éthyl]éthylamino]propyl]-1-(4-fluorophényl)-1,3-dihydroisobenzofurane-5-carbonitrile,
le 1-[3-[[2-(5-chloro-1H-indol-3-yl)éthyl]éthylamino]propyl]-1-(4-fluorophényl)-1,3-dihydroisobenzofurane-5-carbonitrile,
le 1-[3-[[2-(5,7-difluoro-1H-indol-3-yl)éthyl]éthylamino]propyl]-1-(4-fluorophényl)-1,3-dihydroisobenzofurane-5-carbonitrile,
le 1-[3-[[4-(5-fluoro-1H-indol-3-yl)butyl]éthylamino]propyl]-1-(4-fluorophényl)-1,3-dihydroisobenzofurane-5-carbonitrile,
le 1-[3-[[4-(5-chloro-1H-indol-3-yl)butyl]méthylamino]propyl]-1-(4-fluorophényl)-1,3-dihydroisobenzofurane-5-carbonitrile,
le 1-[3-[[3-(5-chloro-1H-indol-3-yl)propyl]éthy]amino]propyl]-1-(4-fluorophényl)-1,3-dihydroisobenzofurane-5-carbonitrile,
le 1-[3-[[3-(5,7-difluoro-1H-indol-3-yl)propyl]éthylamino]propyl]-1-(4-fluorophényl)-1,3-dihydroisobenzofurane-5-carbonitrile,
le 1-[3-[[2-(5-bromo-1H-indol-3-yl)éthyl]méthylamino]propyl]-1-(4-fluorophényl)-1,3-dihydroisobenzofurane-5-carbonitrile,
le 1-[3-[[3-(5-bromo-1H-indol-3-yl)propyl]méthylamino]propyl]-1-(4-fluorophényl)-1,3-dihydroisobenzofurane-5-carbonitrile,
le 1-[3-[[2-(5-bromo-1H-indol-3-yl)éthyl]éthylamino]propyl]-1-(4-fluorophényl)-1,3-dihydroisobenzofurane-5-carbonitrile,
le 1-[3-[[4-(5-bromo-1H-indol-3-yl)butyl]méthylamino]propyl]-1-(4-fluorophényl)-1,3-dihydroisobenzofurane-5-carbonitrile,
le 1-[3-[[3-(5-bromo-1H-indol-3-yl)propyl]méthylamino]propyl]-1-(4-fluorophényl)-1,3-dihydroisobenzofurane-5-carbonitrile,
le 1-[3-[éthyl[2-(5-iodo-1H-indol-3-yl)éthyl]amino]propyl]-1-(4-fluorophényl)-1,3-dihydroisobenzofurane-5-carbonitrile,
le 1-[3-[éthyl[3-(5-iodo-1H-indol-3-yl)propyl]amino]propyl]-1-(4-fluorophényl)-1,3-dihydroisobenzofurane-5-carbonitrile,
le 1-[2-[[4-(5-chloro-1H-indol-3-yl)butyl]méthylamino]éthyl]-1-(4-fluorophényl)-1,3-dihydroisobenzofurane-5-carbonitrile,
le 1-[2-[[4-(5-bromo-1H-indol-3-yl)butyl]méthylamino]éthyl]-1-(4-fluorophényl)-1,3-dihydroisobenzofurane-5-carbonitrile,
le 1-[4-[[2-(5,7-difluoro-1H-indol-3-yl)éthyl]méthylamino]butyl]-1-(4-fluorophényl)-1,3-dihydroisobenzofurane-5-carbonitrile,
le 1-[4-[[2-(7-chloro-1H-indol-3-yl)éthyl]méthylamino]butyl]-1(4-fluorophényl)-1,3-dihydroisobenzofurane-5-carbonitrile,
le 1-[4-[[2-(5-chloro-1H-indol-3-yl)éthyl]méthylamino]butyl]-1-(4-fluorophényl)-1,3-dihydroisobenzofurane-5-carbonitrile,
le 1-[4-[[2-(5-bromo-1H-indol-3-yl)éthyl]méthylamino]butyl]-1-(4-fluorophényl)-1,3-dihydroisobenzofurane-5-carbonitrile,
le 1-[4-[[2-(5-méthyl-1H-indol-3-yl)éthyl]méthylamino]butyl]-1-(4-fluororophényl)-1,3-dihydroisobenzofurane-5-carbonitrile,
le 1-[4-[[2-(5-iodo-1H-indol-3-yl)éthl]méthylamino]butyl]-1-(4-fluorophényl)-1,3-dihydroisobenzofurane-5-carbonitrile,
le 1-[4-[[2-(5-t-butyl-1H-indol-3-yl)éthyl]méthylamino]butyl]-1-(4-fluorophényl)-1,3-dihydroisobenzofurane-5-carbonitrile,
le 1-[4-[[2-(5-i-propyl-1H-indol-3-yl)éthyl]méthylamino]butyl]-1-(4-fluorophényl)-1,3-dihydroisobenzofurane-5-carbonitrile,
le 1-[3-[[2-(5-méthyl-1H-indol-3-yl)éthyl](prop-2-én-1-yl)amino]propyl]-1-(4-fluorophényl)-1,3-dihydroisobenzofurane-5-carbonitrile,
le 1-[3-[[2-(5-fluoro-1H-indol-3-yl)éthyl](prop-2-én-1-yl)amino]propyl]-1-(4-fluorophényl)-1,3-dihydroisobenzofurane-5-carbonitrile,
le 1-[3-[[2-(7-fluoro-1H-indol-3-yl)éthyl](prop-2-én-1-yl)amino]propyl]-1-(4-fluorophényl)-1,3-dihydroisobenzofurane-5-carbonitrile,
le 1-[3-[[3-(5-fluoro-1H-indol-3-yl)propyl](prop-2-én-1-yl)amino]propyl]-1-(4-fluorophényl)-1,3-dihydroisobenzofurane-5-carbonitrile,
le 1-[3-[[3-(7-fluoro-1H-indol-3-yl)propyl](prop-2-én-1-yl)amino]propyl]-1-(4-fluorophényl)-1,3-dihydroisobenzofurane-5-carbonitrile,
le 1-[3-[[2-(5-chloro-1H-indol-3-yl)éthyl](prop-2-én-l-yl)amino]propyl]-1-(4-fluorophényl)-1,3-dihydroisobenzofurane-5-carbonitrile,
le 1-[3-[[2-(5,7-difluoro-1H-indol-3-yl)éthyl]propylamino]propyl]-1-(4-fluorophényl)-1,3-dihydroisobenzofurane-5-carbonitrile,
le 1-[3-[[2-[5-(2-propyl)-1H-indol-3-yl]éthyl]-2-propylamino]propyl]-1-(4-fluorophényl)-1,3-dihydroisobenzofurane-5-carbonitrile,
le 1-[3-[[3-(4-fluoro-7-méthyl-1H-indol-3-yl)propyl](prop-2-én-1-yl)amino]propyl]-1-(4-fluorophényl)-1,3-dihydroisobenzofurane-5-carbonitrile,
le 1-[3-[[2-(4-chloro-7-méthyl-1H-indol-3-yl)éthyl](prop-2-én-1-yl)amino]propyl]-1-(4-fluorophényl)-1,3-dihydroisobenzofurane-5-carbonitrile,
le 1-[3-[[3-(5-chloro-1H-indol-3-yl)propyl](prop-2-én-1-yl)amino]propyl]-1-(4-fluorophényl)-1,3-dihydroisobenzofurane-5-carbonitrile,
le 1-[3-[[2-(5-pyrrolo[3,2-h]-1H-quinolin-3-yl)éthyl](prop-2-én-1-yl)amino]propyl]-1-(4-fluorophényl)-1,3-dihydroisobenzofurane-5-carbonitrile,
le 1-[3-[[3-(7-fluoro-1H-indol-3-yl)propyl](2-furylméthyl)amino]propyl]-1-(4-fluorophényl)-1,3-dihydroisobenzofurane-5-carbonitrile,
le 1-[3-[[4-(7-carboxy-1H-indol-3-yl)butyl](prop-2-én-1-yl)amino]propyl]-1-(4-fluorophényl)-1,3-dihydroisobenzofurane-5-carbonitrile,
le 1-[3-[[2-[5-bromo-1H-indol-3-yl]éthyl]propylamino]propyl]-1-(4-fluorophényl)1,3-dihydroisobenzofurane-5-carbonitrile,
le 1-[3-[[3-(1H-indol-3-yl)propyl](2-phénoxyéthyl)amino]propyl]-1-(4-fluorophényl)-1,3-dihydroisobenzofurane-5-carbonitrile,
le 1-[3-[[2-(5-méthyl-1H-indol-3-yl)éthyl](2-phénoxyéthyl)amino]propyl]-1-(4-fluorophényl)-1,3-dihydroisobenzofurane-5-carbonitrile,
le 1-[3-[[2-(5-fluoro-1H-indol-3-yl)éthyl](2-phénoxyéthyl)amino]propyl]-1-(4-fluorophényl)-1,3-dihydroisobenzofurane-5-carbonitrile,
le 1-[3-[[3-(5-pyrrolo[3,2-h]-1H-quinolin-3-yl)propyl]-2-furylméthylamino]propyl]-1-(4-fluorophényl)-1,3-dihydroisobenzofurane-5-carbonitrile,
le 1-[3-[[3-(5-méthyl-1H-indol-3-yl)propyl](2-phénoxyéthyl)amino]propyl]-1-(4-fluorophényl)-1,3-dihydroisobenzofurane-5-carbonitrile,
le 1-[3-[[3-(5-fluoro-1H-indol-3-yl)propyl](2-phénoxyéthyl)amino]propyl]-1-(4-fluorophényl)-1,3-dihydroisobenzofurane-5-carbonitrile,
le 1-[3-[[2-(5,7-difluoro-1H-indol-3-yl)éthyl](2-phénoxyéthyl)amino]propyl]-1-(4-fluorophényl)-1,3-dihydroisobenzofurane-5-carbonitrile,
le 1-[3-[[4-(5-pyrrolo[3,2-h]-1H-quinolin-3-yl)butyl]-2-furylméthylamino]propyl]-1-(4-fluorophényl)-1,3-dihydroisobenzofurane-5-carbonitrile,
le 1-[3-[2-phénoxyéthyl[2-[5-(2-propyl)-1H-indol-3-yl]éthyl]amino]propyl]-1-(4-fluorophényl)-1,3-dihydroisobenzofurane-5-carbonitrile ou
le 1-[3-[[2-(5-bromo-1H-indol-3-yl)éthyl](2-phénoxyéthyl)amino]propyl]-1-(4-fluorophényl)-1,3-dihydroisobenzofurane-5-carbonitrile,
ou un de ses sels d'addition d'acide.

14. Composition pharmaceutique comprenant un composé selon les revendications 1 à 13 ou un de ses sels d'addition d'acide pharmaceutiquement acceptables et au moins un support ou diluant pharmaceutiquement acceptable.

15. Utilisation d'un composé selon les revendications 1 à 14 ou d'un de ses sels d'addition d'acide pharmaceutiquement acceptables pour la préparation d'un médicament destiné au traitement d'un trouble ou d'une maladie sensible à l'effet des récepteurs 5-HT_{1A}.

16. Utilisation d'un composé selon la revendication 15, dans laquelle le médicament est destiné au traitement d'une dépression, d'une psychose, de troubles liés à l'anxiété, de la panique, de troubles obsessionnels compulsifs, du trouble du contrôle des impulsions, de l'alcoolisme, de l'agressivité, de l'ischémie, de la démence sénile, de maladies cardiovasculaires et de la phobie des contacts sociaux.
